# EUROPEAN PATENT APPLICATION

(11) **EP 4 108 258 A1**
(43) Date of publication of application: **28.12.2022**
(21) Application number: 21756245.3
(22) Date of filing: 19.02.2021
(51) Int. Cl.: A61K 39/395, C07K 16/24

(54) **ANTI-TNF-ALPHA ANTIBODY PREPARATION, PREPARATION METHOD THEREFOR AND USE THEREOF**

(30) Priority: 20.02.2020 CN 202010105359; 31.12.2020 WO PCT/CN2020/141907
(71) Applicant: Bio-Thera Solutions, Ltd., Guangzhou, Guangdong 510530 (CN)
(72) Inventor: YUE, Haitao, Guangzhou, Guangdong 510530 (CN); LIN, Jian, Guangzhou, Guangdong 510530 (CN); WU, Yong, Guangzhou, Guangdong 510530 (CN); WANG, Shengwu, Guangzhou, Guangdong 510530 (CN); LI, Shengfeng, Guangzhou, Guangdong 510530 (CN)
(74) Representative: Aera A/S
(86) International application number: PCT/CN2021/076786
(87) International publication number: WO 2021/164717

(57) **Abstract**

The present invention relates to the field of pharmacy, and provides an anti-TNF-a antibody formulation, and a preparation method and use thereof, wherein the formulation comprises an anti-TNF-a antibody and a pharmaceutically acceptable carrier, the anti-TNF-a antibody comprises a heavy chain with an amino acid sequence set forth in SEQ ID NO: 1 and a light chain with an amino acid sequence set forth in SEQ ID NO: 2, and the pharmaceutically acceptable carrier comprises a stabilizer, a surfactant and a buffer. The antibody formulation of the present invention has high stability and does not support microorganism growth.

## Description

### TECHNICAL FIELD

The present invention belongs to the field of pharmacy, and in particular relates to tumor necrosis factor-a (TNF-α) antibody formulation, and a preparation method and use thereof.

### BACKGROUND

Tumor necrosis factor α (TNF-α), mainly secreted by macrophages and monocytes, is an important pro-inflammatory factor and immune regulator, regulating various physiological processes, such as inducing adhesion molecule expression, regulating lymphocyte to form lymphoid tissue and regulating immune defense in an organism. Studies have found that TNF-α, when present at a low concentration, is considered beneficial to humans, such as enhancing immune responses; while when present at a high concentration, can cause inflammatory reactions and organ damage, for example, the massive production of TNF-α in patients with sepsis in a short term may lead to shock in the patients. Studies have found that TNF-α induces the expression of adhesion molecules, such as intercellular adhesion molecule 1 (ICAM-1) and vascular cell adhesion molecule 1 (VCAM-1), by binding to tumor necrosis factor receptor (TNFR) on the surface of downstream effector cells, so as to aggregate neutrophils, regulate the proliferation, maturation and activation of neutrophils and macrophages, and further stimulate monocytes and vascular endothelial cells to release other cytokines such as the pro-inflammatory factors interleukin factor 1 (IL-1) and interleukin factor 8 (IL-8), forming a cascade reaction and eventually leading to inflammatory damage of the tissue (Paul A T, Gohil V M, Bhutani K K. Modulating TNF-α signaling with natural products. Drug Discovery Today, 2006, 11(11):725-32.). TNF-α with an increased concentration is found in the blood and articular synovium of a number of patients with chronic inflammatory diseases such as psoriatic arthritis, rheumatoid arthritis, ankylosing spondylitis and the like, indicating that TNF-α is closely associated with these diseases. Many newly developed biologics have recently been able to control disease progression by blocking or down-regulating the activity of TNF-α, such as TNF-α inhibitors. The main mechanism of action of TNF-α inhibitors is that after specifically binding to secretory tumor necrosis factor α (sTNF-α), the TNF-α inhibitors can block sTNF-α binding to TNFR and inhibit biological activity of the sTNF-α, and the TNF-α inhibitors can bind to transmembrane tumor necrosis factor α (tmTNF-α) on TNF-α synthetic cells and activate a reverse signaling pathway to reduce the synthesis amount of the TNF-α and even cause the apoptosis of the TNF-α synthetic cells, thereby relieving the inflammatory symptoms of patients.

Currently, commercially available TNF-α inhibitor drugs mainly comprise Infliximab (Remicade^{®}), Etanercept (Enbrel^{®}), Adalimumab (Humira^{®}), Golimumab (Simponi^{®}) and Certolizumab pegol (Cimzia^{®}). Golimumab, a fully human monoclonal antibody, is approved by NDA (New Drug Application) as a subcutaneous injection formulation, and is the first internationally marketed TNF-α inhibitor drug that can be subcutaneously administered once every 4 weeks.

The immunogenicity of the currently widely used fully human antibody is greatly reduced compared to that of the original murine antibody. However, mammalian cells can synthesize Neu5Gc sialic acid (NGNA) which is immunogenic to humans, for example, when Sp2/0 cells are used for antibody production, antibody has a higher NGNA glycosylation level, which may cause the risk of human immune response.

Accordingly, there is still a need in field for anti-TNF-a antibodies formulations with improved properties.

### SUMMARY OF THE INVENTION

In a first aspect, the present invention provides an anti-TNF-a antibody product. In some embodiments, the anti-TNF-a antibody product is an anti-TNF-a antibody formulation. The formulation has improved performance, and has excellent antibody protein stability and formulation stability (especially, the excellent antibody protein stability is still maintained under the conditions of high temperature, illumination change, repeated freeze-thaw and the like) under high-concentration antibody protein, thereby reducing the risk of adverse effects and avoiding the growth of microorganisms. The formulation comprises an anti-TNF-a antibody and a pharmaceutically acceptable carrier.

In some embodiments, the anti-TNF-a antibody comprises a heavy chain with an amino acid sequence set forth in SEQ ID NO: 1 and a light chain with an amino acid sequence set forth in SEQ ID NO: 2.

In some embodiments, the pharmaceutically acceptable carrier comprises a stabilizer; in some embodiments, the stabilizer is a sugar, arginine hydrochloride, glycine, methionine or a combination thereof; in some embodiments, the stabilizer is trehalose or sucrose; in some embodiments, the stabilizer is trehalose. In some embodiments, the antibody formulation does not comprise a sugar alcohol.

In some embodiments, the concentration of the anti-TNF-a antibody is 5-130 mg/mL; in some embodiments, the concentration of the anti-TNF-a antibody is 50-130 mg/mL; in some embodiments, the concentration of the anti-TNF-a antibody is 90-110 mg/mL; in some embodiments, the concentration of the anti-TNF-a antibody is 100 mg/mL.

In some embodiments, the concentration of the stabilizer is 160-265 mM; in some embodiments, the concentration of the stabilizer is 210-240 mM; in some embodiments, the concentration of the stabilizer is 225 mM. In some embodiments, the stabilizer is trehalose at 225 mM.

In some embodiments, the antibody formulation may be in a liquid form, a powder form or any other suitable form. The powder form may be resuspended into a solution form before use according to administration route, storage conditions and the like.

In some embodiments, the pharmaceutically acceptable carrier further comprises a surfactant and/or a buffer.

In some embodiments, the surfactant is a polysorbate; in some embodiments, the surfactant is a polysorbate 20 and/or a polysorbate 80; in some embodiments, the surfactant is a polysorbate 80; in some embodiments, the concentration of the surfactant is 0.05-0.5 mg/mL; in some embodiments, the concentration of the surfactant is 0.1-0.5 mg/mL; in some embodiments, the concentration of the surfactant is 0.1-0.3 mg/mL; in some embodiments, the concentration of the surfactant is 0.2 mg/mL. In some embodiments, the surfactant is a polysorbate 80 at 0.2 mg/mL.

In some embodiments, the pH of the antibody formulation is 4.5-6.5; in some embodiments, the pH thereof is 4.5-6.0; in some embodiments, the pH thereof is 5.0-6.0; in some embodiments, the pH thereof is 5.0-5.8; in some embodiments, the pH thereof is 5.2-5.8; in some embodiments, the pH thereof is 5.5.

In some embodiments, the antibody formulation comprises a solvent. In some embodiments, the solvent is, e.g., water, such as water for injection.

In some embodiments, the buffer in the antibody formulation is one or more of a histidine buffer, a glutamine buffer, a sodium acetate buffer, a succinate buffer and a citrate buffer; in some embodiments, the buffer is a histidine buffer, a glutamine buffer and a sodium acetate buffer; in some embodiments, the buffer is a histidine buffer. In some embodiments, the concentration of the buffer is 5-30 mM; in some embodiments, the concentration of the buffer is 10-20 mM; in some embodiments, the concentration of the buffer is 10 mM. In some embodiments, the buffer is a histidine buffer at 10 mM.

The various pharmaceutically acceptable carriers in the antibody formulation may be provided either together with the antibody or separately and mixed before use.

In some embodiments, an anti-TNF-a antibody formulation is provided that comprises solvent water, an anti-TNF-a antibody, a surfactant, a stabilizer and a buffer; the anti-TNF-a antibody comprises a heavy chain with an amino acid sequence set forth in SEQ ID NO: 1 and a light chain with an amino acid sequence set forth in SEQ ID NO: 2, the antibody formulation comprises the anti-TNF-a antibody at 5-130 mg/mL, the stabilizer at 160-265 mM, the surfactant at 0.05-0.5 mg/mL and the buffer at 5-30 mM, and the pH of the antibody formulation is 4.5-6.5. In some embodiments, the antibody formulation comprises the anti-TNF-a antibody at 90-110 mg/mL, the stabilizer at 210-240 mM, the surfactant at 0.1-0.3 mg/mL and the buffer at 10-20 mM, and the pH of the antibody formulation is 4.5-6.0. In some embodiments, the antibody formulation comprises the anti-TNF-a antibody at 90-110 mg/mL, trehalose at 210-240 mM, a polysorbate 80 at 0.1-0.3 mg/mL and a histidine buffer at 10-20 mM, and the pH of the antibody formulation is 4.5-6.0. In some embodiments, the antibody formulation comprises the anti-TNF-a antibody at 100 mg/mL, trehalose at 225 mM, a polysorbate 80 at 0.2 mg/mL and a histidine buffer at 10 mM, and the pH of the antibody formulation is 5.0-5.8.

In some embodiments, an anti-TNF-a antibody formulation is provided that comprises solvent water, an anti-TNF-a antibody, a surfactant, a stabilizer and a buffer; the anti-TNF-a antibody comprises a heavy chain with an amino acid sequence set forth in SEQ ID NO: 1 and a light chain with an amino acid sequence set forth in SEQ ID NO: 2, the concentration of the anti-TNF-a antibody is 100 mg/mL, the stabilizer is trehalose dihydrate at 85 mg/mL, the surfactant is a polysorbate 80 at 0.2 mg/mL, the antibody formulation has a pH value of pH 5.5, and the pH value is maintained by adding histidine hydrochloride at 1.72 mg/mL and histidine at 0.28 mg/mL to the liquid composition of the antibody formulation.

In a second aspect, the present invention provides a method for preparing the anti-TNF-a antibody formulation described as above.

In one embodiment, provided is a method for preparing the liquid anti-TNF-a antibody formulation described as above, the method comprises:
weighing a stabilizer, a surfactant and a buffer,
dispersing the weighed ingredients in a liquid solvent for injection to prepare a solvent system, and
mixing the solvent system with the anti-TNF-a antibody under agitation.

In some embodiments, the buffer may be one or more of a histidine buffer, a glutamine buffer, a sodium acetate buffer, a succinate buffer and a citrate buffer; the stabilizer may be a sugar, arginine hydrochloride, glycine, methionine or a combination thereof; the surfactant may be a polysorbate.

In some embodiments, provided is a method for preparing an anti-TNF-a antibody formulation, the method comprises:
weighing trehalose, a polysorbate 80, histidine and histidine hydrochloride;
dissolving the weighed ingredients in water for injection to prepare a solvent system; and mixing the solvent system with the anti-TNF-a antibody under agitation;
so that the final concentrations of ingredients in the antibody formulation including anti-TNF-a antibody, trehalose, polysorbate 80, histidine hydrochloride and histidine are 90-110 mg/mL, 80-90 mg/mL, 0.1-0.3 mg/mL, 1-3 mg/mL and 0.2-0.5 mg/mL, respectively, and the final pH of the antibody formulation is 5.0-6.0.

In some embodiments, the final concentrations of ingredients in the antibody formulation including anti-TNF-a antibody, trehalose, polysorbate 80, histidine hydrochloride and histidine are 100 mg/mL, 85 mg/mL, 0.2 mg/mL, 1.72 mg/mL and 0.28 mg/mL, respectively.

The method may further comprise the steps of preparing an anti-TNF-a antibody formulation and/or sterilizing and packaging the antibody formulation. For example, the prepared antibody formulation is sterilized by passing through a hydrophilic poly(vinylidene fluoride) or polyethersulfone filter with a pore diameter of 0.22 µm.

In a third aspect, the present invention provides a method for treating a TNF-α related diseases comprising administering to a patient in need thereof the anti-TNF-a antibody formulation of the present invention described as above. The TNF-α related diseases include, but are not limited to, chronic inflammatory diseases such as psoriatic arthritis, rheumatoid arthritis, ankylosing spondylitis and the like.

In some embodiments, the method comprises administering the antibody formulation of the present invention by subcutaneous injection; in some embodiments, the administration is performed once every 4 weeks; in some embodiments, administration amount is 50 mg.

In a fourth aspect, the present invention provides an antibody pharmaceutical product for treating a TNF-α related disease comprising the anti-TNF-a antibody formulation of the present invention described as above and a container for storing the formulation. The pharmaceutical product may also include instructions for use. The container may be any container conventionally used in the art for storing drug, such as pre-filled containers, pre-filled syringes, vials, ampoules, sachets and the like.

In a fifth aspect, the present invention provides use of the anti-TNF-a antibody formulation or antibody pharmaceutical product of the present invention described as above in the formulation of a medicament for treating a TNF-α related disease. The TNF-α related diseases include, but are not limited to, chronic inflammatory diseases such as psoriatic arthritis, rheumatoid arthritis, ankylosing spondylitis, ulcerative colitis and the like.

In a sixth aspect, the present invention provides an anti-TNF-a antibody with a low Neu5Gc sialic acid (NGNA) level, comprising a heavy chain with an amino acid sequence set forth in SEQ ID NO: 1 and a light chain with an amino acid sequence set forth in SEQ ID NO: 2, for reducing the risk of adverse effects without affecting antibody activity. In some embodiments, the molar ratio of the Neu5Gc sialic acid (NGNA) in the anti-TNF-a antibody to the anti-TNF-a antibody is no more than 0.1. In some embodiments, the anti-TNF-a antibody is expressed by a CHO cell containing a nucleic acid or vector encoding the anti-TNF-a antibody. In some embodiments, the CHO cell is a CHO-K1 cell. In some embodiments, the CHO-K1 cell is acclimated to a CHO-K1 cell suitable for suspension culture.

In a seventh aspect, the present invention provides a method for preparing the anti-TNF-a antibody of the present invention. In some embodiments, the methods use a CHO cell line as a host cell. In some embodiments, the method comprises: culturing a CHO cell containing a nucleic acid or vector encoding the anti-TNF-a antibody such that the CHO cell expresses the anti-TNF-a antibody. In some embodiments, the method comprises following steps of: 1) transfecting a CHO cell with a nucleic acid or vector encoding the anti-TNF-a antibody; 2) culturing the CHO cell such that the CHO cell expresses the anti-TNF-a antibody; in some embodiments, the anti-TNF-a antibody comprises a heavy chain with an amino acid sequence set forth in SEQ ID NO: 1 and a light chain with an amino acid sequence set forth in SEQ ID NO: 2. In some embodiments, the CHO cell is a CHO-K1 cell.

In some embodiments, the method further comprises the steps of collecting culture medium after stopping culturing. In some embodiments, the method further comprises purifying the anti-TNF-a antibody after collecting the culture medium.

In the seventh aspect, the present invention provides the anti-TNF-a antibody having a reduced level of deamidation and/or a reduced level of NGNA glycosylation prepared by the foregoing method. In some embodiments, the molar ratio of the Neu5Gc sialic acid (NGNA) in the anti-TNF-a antibody to the anti-TNF-a antibody is no more than 0.1; in some embodiments, the molar ratio of NGNA in the anti-TNF-a antibody to the anti-TNF-a antibody is no more than 0.01; in some embodiments, the molar ratio of NGNA in the anti-TNF-a antibody to the anti-TNF-a antibody is no more than 0.005.

In some embodiments, the anti-TNF-a antibody expressed by the CHO cell comprises a heavy chain amino acid sequence set forth in SEQ ID NO: 1 and a light chain amino acid sequence set forth in SEQ ID NO: 2, and the anti-TNF-a antibody is substantially identical (differs by no more than ± 20%, or ± 10% or ± 5%, or no more than 3-fold, 2-fold or 1-fold of standard deviation) to Simponi under one or more of the relative affinity for binding to TNF-α, the ability to bind to TNF-α, the inhibitory effect on the biological activity of TNF-α, the relative affinity for binding to FcRn, the relative affinity for binding to FcyRIIIa (F158), the relative affinity for binding to FcyRIIIa (V158) and the pharmacokinetic characteristics in cynomolgus monkeys (e.g., plasma drug concentration). In some embodiments, the anti-TNF-a antibody expressed by the CHO cell is Antibody A.

In an eighth aspect, the present invention provides an antibody formulation comprising the anti-TNF-a antibody described as above. In some embodiments, the antibody formulation further comprises a pharmaceutically acceptable carrier.

With antibody activity ensured, the NGNA glycosylation level of the anti-TNF-a antibody developed by the present invention is low, ensuring higher antibody activity and lower immunogenicity. The anti-TNF-a antibody formulation developed by the present invention comprises the high-concentration anti-TNF-a antibody protein of the present invention with higher antibody activity and lower immunogenicity, and the high-concentration antibody protein contained in the formulation is stable, particularly, the formulation can resist protein aggregation reaction caused by high temperature, illumination and repeated freeze-thaw, and avoids the growth of microorganisms. Accordingly, the anti-TNF-a antibody formulations of the present invention meet the need in the art for anti-TNF-a antibody pharmaceutical formulations with improved performance.

### BRIEF DESCRIPTION OF THE DRAWINGS

FIG. 1 depicts the results of size-exclusion chromatography in screening for the buffer in the anti-TNF-a antibody formulation in Example 3.
FIG. 2 depicts the results of capillary electrophoresis in the stability of the anti-TNF-a antibody formulation of the present invention at high temperature (40 °C).
FIG. 3 depicts the results of size-exclusion chromatography in a freeze-thaw stability of the anti-TNF-a antibody formulation of the present invention.
FIG. 4 depicts the relative affinities of the Antibody A formulation of the present invention and Simponi binding to TNF-α.
FIG. 5 depicts the relative binding capacity of the Antibody A formulation of the present invention and Simponi binding to TNF-α.
FIG. 6 depicts the inhibitory effect of the Antibody A formulation of the present invention and Simponi on the biological activity of TNF-α.
FIG. 7A depicts the effect of Antibody A of the present invention on the arthritis scoring for Tg197 transgenic spontaneous arthritis mouse model.
FIG. 7B depicts the effect of Antibody A of the present invention on histopathological scoring for Tg197 transgenic spontaneous arthritis mouse model.
FIGs. 8A-8B depict a molecular weight comparison of Antibody A of the present invention and Simponi (FIG. 8A: intact molecular weight; FIG. 8B: desugared molecular weight).
FIG. 9 depicts the relative affinities of Antibody A of the present invention and Simponi binding to FcRn.
FIG. 10 depicts the relative affinities of Antibody A of the present invention and Simponi binding to FcyRIIIa (F158).
FIG. 11 depicts the relative affinities of Antibody A of the present invention and Simponi binding to FcyRIIIa (V158).
FIG. 12A depicts a pharmacokinetic comparison between Antibody A of the present invention and Simponi both at a low dose of 3 mg/kg in cynomolgus monkeys.
FIG. 12B depicts a pharmacokinetic comparison between Antibody A of the present invention and Simponi both at a high dose of 10 mg/kg in cynomolgus monkeys.

### DETAILED DESCRIPTION OF THE INVENTION

In order to more clearly understand the technical contents of the present invention, the following examples are given in detail. It should be understood that the examples are merely intended to illustrate the present invention rather than limit the scope of the present invention. Those skilled in the art will recognize or be able to recognize various equivalent specific embodiments of the present invention only by routine experimentation. Such equivalent embodiments are included within the scope of the present invention. All documents and patents cited herein are incorporated by reference. Experimental processes without specific conditions noted in the following examples generally depend on conventional conditions or conditions recommended by manufacturers. The various common chemical reagents used in the examples are commercially available.

It will be understood by those skilled in the art that the weights and/or weight to volume ratios referred to herein may be converted to molar and/or molar concentrations using the well known molecular weights of respective ingredients. The weights listed in the examples are for respective volumes. It will be understood by those skilled in the art that where different formulation volumes are required, the weights may be adjusted proportionally. Unless defined otherwise, all technical and scientific terms used herein have the same meaning as commonly understood by those skilled in the art to which the present invention belongs. The terms used in the specification of the present invention herein is for the purpose of describing specific embodiments only and is not intended to limit the scope of the present invention. As used herein, the term "and/or" includes any and all combinations of one or more of the associated listed items.

### Definitions

As used herein, "%" ingredient in the present invention specifically refers to weight volume (w/v) percent, wherein the weight unit may be g and the volume unit may be mL. For example, 1% stabilizer in a solution represents 1 g stabilizer in 100 mL solution or stabilizer at 0.01 g/mL.

"About" or "approximately" refers to an ordinary deviation range for corresponding values as readily understood by those skilled in the relevant art. In some embodiments, "about" or "approximately" mentioned herein refer to the numerical values described as well as its ranges of ± 10%, ± 5%, ± 1% or ± 0.1%.

"Comprising" or "including" means that the composition, method and the like include the listed elements (e.g., ingredients of the compositions, steps of the methods, and the like), but do not exclude others. When used to define compositions or methods, "consisting essentially of" means excluding elements that have a material affect on the combination for its intended use, but does not exclude elements that do not materially affect the characteristics of the composition or method. "Consisting of" shall mean excluding elements not specifically recited. Embodiments defined by each of these transition terms are within the scope of this invention. For example, when a composition is described as comprising ingredients A, B and C, a composition consisting essentially of A, B and C and a composition consisting of A, B and C is independently within the scope of the present invention.

As used herein, "antibody" refers to a polypeptide or polypeptide complex that specifically recognizes and binds to an antigen. The antibody may be an intact antibody and any antigen-binding fragment or single chain thereof. Thus, the term "antibody" includes any protein or peptide comprising a molecule that comprises at least a portion of an immunoglobulin molecule having biological activity that binds to an antigen. Such examples include, but are not limited to, complementarity determining regions (CDRs) of a heavy or light chain or ligand binding portion thereof, heavy or light chain variable regions, heavy or light chain constant regions, framework (FR) regions or any portion thereof, or at least a portion of a binding protein.

As used herein, the term "treatment" refers to both therapeutic treatment and prophylactic measures, the purpose of which is to prevent or slow down (reduce) the progression of an undesired physiological change or disease, such as a TNF-α related disease. Beneficial or desired clinical results include, but are not limited to, alleviating symptoms, reducing the severity of disease, stabilizing (i.e., not worsening) disease progression, delaying or slowing disease progression, improving or alleviating disease state, and eradicating disease (whether partial or total), whether detectable or undetectable. Those in need of treatment include patients already with the condition or disease, as well as patients susceptible to the condition or disease, or patients in need of prevention of the condition or disease.

As used herein, "patients" refer to any subject, particularly mammalian subjects in need of treatment. Mammalian subjects include humans, domestic animals, livestock, zoo animals, sports animals, or pet animals, such as dogs, cats, guinea pigs, rabbits, rats, mice, horses, cows and the like.

As used herein, "patients in need thereof" include patients who would benefit from administration of the antibody or formulation of the present invention, such as mammalian patients.

The term "pharmaceutically acceptable" as used herein refers to a drug listed in the generally recognized pharmacopoeia that can be used in animals, particularly in humans. "A pharmaceutically acceptable carrier" is typically a non-toxic solid, semi-solid or liquid filler, diluent, encapsulating material or any type of formulation additive.

The term "buffer", also referred as a buffer system in some literature, includes, but is not limited to, organic acid salts such as succinic acid, acetic acid, citric acid, ascorbic acid, gluconic acid, carbonic acid, tartaric acid or phthalic acid and salts thereof; Tris; thomethamine hydrochloride or phosphate buffer. In addition, amino acids and salts thereof may also be used as buffers. Such amino acid ingredients include, but are not limited to, glycine, histidine, arginine, lysine, ornithine, isoleucine, leucine, alanine, glutamic acid or aspartic acid. In some embodiments, the buffer is a histidine salt buffer.

The amount of the buffer in the present invention means the total amount of the buffer pair in the buffer system forming the buffer. In some embodiments, molar concentration is taken as a unit of the amount of the buffer, the value of which refers to the molar concentration of the buffer pair in the buffer system of the buffer. For example, where histidine buffer consisting of histidine and histidine hydrochloride is used as the buffer, a given concentration of histidine salt buffer (e.g., 10 mM) is the combined concentration of histidine and histidine hydrochloride (e.g., histidine at 5 mM and histidine hydrochloride at 5 mM; or histidine at 1.8 mM and histidine hydrochloride at 8.2 mM).

The term "stabilizer" includes, but is not limited to, monosaccharides such as fructose, maltose, galactose, glucose, sorbose and the like; disaccharides such as lactose, sucrose, trehalose, cellobiose and the like; polysaccharides such as raffinose, melezitose, maltodextrin, dextran, starch and the like; ionic stabilizers include salts such as NaCl or amino acid ingredients such as arginine-HCl, proline, glycine and methionine.

The term "surfactant" includes, but is not limited to, polysorbates (such as polysorbate 20 and polysorbate 80), poloxamers (e.g., poloxamer 188), triton, sodium dodecyl sulfate (SDS), sodium lauryl sulfate; sodium octyl glycoside, lauryl sulfobetaine, myristyl sulfobetaine, linoleyl sulfobetaine or stearyl sulfobetaine, lauryl sarcosine, myristyl sarcosine, linoleyl sarcosine or stearyl sarcosine, linoleyl betaine, myristyl betaine or cetyl betaine, lauryl amidopropyl betaine, cocoamidopropyl betaine, linoleamidopropyl betaine, myristamidopropyl betaine, palmitamidopropyl betaine or isostearamidopropyl betaine (e.g. laurylamidopropyl), myristamidopropyl dimethylamine, palmitoylaminopropyl dimethylamine or isostearamidopropyl dimethylamine, sodium methyl cocoyl taurate or disodium methyl oleyl taurate, polyethylene glycols, polypropylene glycols, copolymers of ethylene and propylene glycols (e.g., Pluronics, PF68, etc.) and the like. In some embodiments, the surfactant is polysorbate 80.

The term "pharmaceutically acceptable" refers to materials for animals, particularly humans, which are approved by a regulatory authority or listed in the *Chinese Pharmacopoeia* or other commonly-recognized pharmacopoeias. In addition, the "pharmaceutically acceptable carrier" will generally may be any type of non-toxic solid, semi-solid or liquid filler, diluent, an encapsulating material, or a formulation additive.

### Anti-TNF-a antibody

The present invention provides an anti-TNF-a antibody with high affinity for TNF-α protein. The present invention discloses an anti-TNF-a antibody comprising a heavy chain and a light chain, wherein the heavy chain amino acid sequence set forth in SEQ ID NO: 1 has 80%, 85%, 90%, 95%, 98% or 99% of identity to the sequence SEQ ID NO: 1, and the light chain amino acid sequence set forth in SEQ ID NO: 2 has 80%, 85%, 90%, 95%, 98% or 99% identity to the sequence SEQ ID NO: 2.
The sequence SEQ ID NO: 1 is: 
QVQLVESGGGVVQPGRSLRLSCAASGFIFSSYAMHWVRQAPGNGLEWVAFMSYDGSN
KKYADSVKGRFTISRDNSKNTLYLQMNSLRAEDTAVYYCARDRGIAAGGNYYYYGMD
VWGQGTTVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDK
 
THTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGK;
The sequence SEQ ID NO: 2 is:

EIVLTQSPATLSLSPGERATLSCRASQSVYSYLAWYQQKPGQAPRLLIYDASNRATGIPA
RFSGSGSGTDFTLTISSLEPEDFAVYYCQQRSNWPPFTFGPGTKVDIKRTVAAPSVFIFPP
SDEQLKSGTASVVCLLNNFYPREAKVQWKVDNALQSGNSQESVTEQDSKDSTYSLSST
LTLSKADYEKHKVYACEVTHQGLSSPVTKSFNRGEC.

In some embodiments, the anti-TNF-a antibody is golimumab (Simponi^{®} or biological analogs thereof (e.g., BOW100, ONS-3035 or Antibody A)).

In some embodiments, the anti-TNF-a antibody is prepared by: culturing a CHO cell transfected with a nucleic acid or vector encoding the anti-TNF-a antibody such that the CHO cell expresses the anti-TNF-a antibody. In some embodiments, the CHO cell is a CHO-K1 cell. In some embodiments, the CHO-K1 cell is a CHO-K1 cell suitable for suspension culture. In some embodiments, the vector may be a plasmid vector, a phage vector, a viral vector, a non-viral vector or a minicircle DNA. In some embodiments, culturing the CHO cell comprises expressing the anti-TNF-a antibody by the CHO cell under appropriate conditions and medium.

In some embodiments, culturing the CHO cell comprises: culturing under suitable conditions and medium for a period of time such that the CHO cells express the heavy and light chains of the antibody and form the anti-TNF-a antibody. In some embodiments, the method further comprises the steps of collecting culture medium after stopping culturing. In some embodiments, the method further comprises purifying the anti-TNF-a antibody after collecting the culture medium.

In some embodiments, the method further comprises isolating the culture medium by centrifugation to obtain the anti-TNF-a antibody after collecting the culture medium. In some embodiments, the isolated anti-TNF-a antibody is purified by conventional means such as affinity chromatography, anion exchange chromatography and/or cation exchange chromatography and the like; in some embodiments, the purity of the purified anti-TNF-a antibody can be greater than about 90%, greater than about 92%, greater than about 94%, greater than about 96%, greater than 98% or greater than about 99%.

Glycosylation is one of the key quality attributes of an antibody. The realization of the function of the monoclonal antibody is closely related to the glycosylation thereof, and the glycosylation modification can influence the performance of protein, such as conformation, stability, solubility, pharmacokinetics, activity and immunogenicity. Glycosylation can be divided into N-glycosylation and O-glycosylation according to the modification site of glycosylation. N-glycosylation in immunoglobulins secreted by animal cells is the most common glycosylation, whereas antibody glycosylation is also mainly N-glycosylation, and for golimumab, glycosylation occurs mostly at Asn-306. According to the differences in the fine structure of glycosylation end (length, branch and monosaccharide arrangement), the glycosylation type can be divided into a compound type, a hybrid type and a high mannose type, and these types are closely related to cell species and cell culture conditions.

The immunogenicity of the currently widely used fully human antibody is greatly reduced compared to that of the original murine antibody. However, mammalian cells can synthesize Neu5Gc sialic acid (NGNA) which is immunogenic to humans, for example, when Sp2/0 cells are used for antibody production, the antibody has a higher NGNA glycosylation level, which may cause the risk of human immune response.

The present invention provides an anti-TNF-a antibody with low NGNA level comprising a heavy chain with an amino acid sequence set forth in SEQ ID NO: 1 and a light chain with an amino acid sequence set forth in SEQ ID NO: 2. In some embodiments, the molar ratio of the Neu5Gc sialic acid (NGNA) in the anti-TNF-a antibody to the anti-TNF-a antibody is no more than 0.1. In some embodiments, the molar ratio of the Neu5Gc sialic acid (NGNA) in the anti-TNF-a antibody to the anti-TNF-a antibody is no more than about 0.1, about 0.09, about 0.08, about 0.07, about 0.06, about 0.05, about 0.04, about 0.03 or about 0.02. In some embodiments, the molar ratio of NGNA in the anti-TNF-a antibody to the anti-TNF-a antibody is no more than about 0.01, no more than about 0.009, no more than about 0.008, no more than about 0.007, no more than about 0.006, no more than about 0.005 or no more than about 0.0045. In some embodiments, the anti-TNF-a antibody is expressed by a CHO cell containing a nucleic acid or vector encoding the anti-TNF-a antibody.

The present invention provides a method for preparing the anti-TNF-a antibody of the present invention, comprising the steps of: preparing a nucleic acid or vector encoding an anti-TNF-a antibody; transfecting CHO-K1 cells with the nucleic acid or vector encoding the anti-TNF-a antibody; expressing the anti-TNF-a antibody by CHO-K1 cells under suitable conditions and medium; stopping culturing; collecting culture medium; isolating the culture medium by centrifugation to obtain the anti-TNF-a antibody; primarily purifying the anti-TNF-a antibody obtained by centrifugation through affinity chromatography; further purifying the primarily purified anti-TNF-a antibody by anion and cation exchange chromatography to obtain the purified anti-TNF-a antibody. In some embodiments, the present invention provides Antibody A; the Antibody A is an anti-TNF-a antibody comprising a heavy chain amino acid sequence set forth in SEQ ID NO: 1 and a light chain amino acid sequence set forth in SEQ ID NO: 2; and Antibody A is prepared by the method as above.

The present invention provides an anti-TNF-a antibody having a lower level of NGNA glycosylation prepared by the method as above. In some embodiments, the molar ratio of the Neu5Gc sialic acid (NGNA) in the anti-TNF-a antibody to the anti-TNF-a antibody is no more than about 0.1, about 0.09, about 0.08, about 0.07, about 0.06, about 0.05, about 0.04, about 0.03 or about 0.02. In some embodiments, the molar ratio of NGNA in the anti-TNF-a antibody to the anti-TNF-a antibody is no more than about 0.01, no more than about 0.009, no more than about 0.008, no more than about 0.007, no more than about 0.006, no more than about 0.005 or no more than about 0.0045.

### Formulation

The antibody formulation of the present invention may be liquid solutions (e.g., injectable, infusible, perfusable solutions), or powders. The form depends on the desired administration method and therapeutic use. The formulations are in the form of injectable, infusible or perfusable solutions. The administration method is parenteral (e.g., intravenous, subcutaneous, intraperitoneal and intramuscular). In some embodiment, the antibody is administered by intravenous perfusion or injection. In some embodiment, the antibody is administered by intramuscular or subcutaneous injection.

Supplementary active compounds can also be incorporated into the pharmaceutical formulations. In some embodiments, the antibody or antigen-binding portion thereof of the present invention is co-formulated and/or co-administered with one or more additional therapeutic agents. For example, the additional therapeutic agents may be a DMARD, an NSAID, other antibodies capable of binding to other targets (e.g., antibodies capable of binding to other cytokine or cell surface molecule), a cytokine, a soluble TNF-α receptor, a chemical inhibiting TNF-α production and activity, or any combination thereof. Furthermore, the antibody of the present invention may be used in combination with the foregoing therapeutic agents. Such combination therapy may advantageously employ lower doses of the administered therapeutic agents, thereby avoiding possible side effects, complications or low levels of patient responses associated with each monotherapy, or increasing the efficacy.

In some embodiments, the antibody formulation is an anti-TNF-a antibody formulation. In some embodiments, the anti-TNF-a antibody comprises a heavy chain with an amino acid sequence set forth in SEQ ID NO: 1 and a light chain with an amino acid sequence set forth in SEQ ID NO: 2. In some embodiments, the concentration of the anti-TNF-a antibody is about 5-130 mg/mL; in some embodiments, the concentration of the anti-TNF-a antibody is about 50-130 mg/mL; in some embodiments, the concentration of the anti-TNF-a antibody is about 90-110 mg/mL; in some embodiments, the concentration of the anti-TNF-a antibody is about 100 mg/mL. In some embodiments, the concentration of the anti-TNF-a antibody is about 5 mg/mL, about 10 mg/mL, about 30 mg/mL, about 50 mg/mL, about 70 mg/mL, about 80 mg/mL, about 90 mg/mL, about 100 mg/mL, about 110 mg/mL, about 120 mg/mL, about 130 mg/mL, or a range between any two concentration values, including end values.

In some embodiments, the anti-TNF-a antibody in the anti-TNF-a antibody formulation is an anti-TNF-a antibody expressed by CHO cells. In some embodiments, the CHO cells expressing the anti-TNF-a antibody are CHO-K1 cells.

In some embodiments, the molar ratio of the NGNA in the anti-TNF-a antibody to the anti-TNF-a antibody is no more than 0.1; in some embodiments, the molar ratio of NGNA in the anti-TNF-a antibody to the anti-TNF-a antibody is no more than 0.01; in some embodiments, the molar ratio of NGNA in the anti-TNF-a antibody to the anti-TNF-a antibody is no more than 0.005.

In some embodiments, the pharmaceutically acceptable carrier comprises a stabilizer; in some embodiments, the stabilizer is a sugar, arginine hydrochloride, glycine, methionine or a combination thereof; in some embodiments, the stabilizer is trehalose or sucrose; in some embodiments, the stabilizer is trehalose. In some embodiments, the concentration of the stabilizer is about 160-265 mM; in some embodiments, the concentration of the stabilizer is about 210-240 mM; in some embodiments, the concentration of the stabilizer is about 225 mM. In some embodiments, the stabilizer is trehalose at about 225 mM. In some embodiments, the stabilizer is trehalose or sucrose, and the concentration of the stabilizer is about 160 mM, about 180 mM, about 200 mM, about 210 mM, about 225 mM, about 240 mM, about 250 mM, about 265 mM, or a range between any two concentration values, including endpoint values.

Trehalose is generally present as trehalose dihydrate in a solid state, so in some embodiments, the formulation is formulated with trehalose dihydrate, or with other forms of trehalose in the corresponding amounts, and the resulting formulation contains the same concentration of trehalose. Where trehalose dihydrate is used herein to describe the amount of trehalose in the formulation, the formulation may contain such an amount of trehalose dihydrate or a corresponding amount of trehalose or other forms of trehalose or combinations thereof, and *vice versa.*

In some embodiments, the concentration of the stabilizer is about 60-100 mg/mL; in some embodiments, the concentration of the stabilizer is about 80-90 mg/mL; in some embodiments, the concentration of the stabilizer is about 85 mg/mL. In some embodiments, the stabilizer is trehalose dihydrate at about 85 mg/mL. In some embodiments, the stabilizer is trehalose dihydrate or sucrose, and the concentration of the stabilizer is about 60 mg/mL, about 70 mg/mL, about 75 mg/mL, about 80 mg/mL, about 85 mg/mL, about 90 mg/mL, about 95 mg/mL, about 100 mg/mL, or a range between any two concentration values, including endpoint values.

In some embodiments, the pharmaceutically acceptable carrier further comprises a surfactant and/or a buffer.

In some embodiments, the formulation further comprises a surfactant. In some embodiments, the surfactant comprises a non-ionic surfactant such as polysorbate 80. The surfactant reduces aggregation of the antibody and/or reduces particle formation in the formulation and/or reduces adsorption. In some embodiments, the surfactant is a polysorbate; in some embodiments, the surfactant is polysorbate 20 and/or polysorbate 80; in some embodiments, the surfactant is polysorbate 80; in some embodiments, the concentration of the surfactant is about 0.05-0.5 mg/mL; in some embodiments, the concentration of the surfactant is about 0.1-0.5 mg/mL; in some embodiments, the concentration of the surfactant is about 0.1-0.3 mg/mL; in some embodiments, the concentration of the surfactant is about 0.2 mg/mL. In some embodiments, the surfactant is a polysorbate 80 at about 0.2 mg/mL. In some embodiments, the concentration of the polysorbate 80 is about 0.05 mg/mL, about 0.1 mg/mL, about 0.15 mg/mL, about 0.2 mg/mL, about 0.25 mg/mL, about 0.3 mg/mL, about 0.4 mg/mL, about 0.5 mg/mL, or a range between any two concentration values, including endpoint values.

In some embodiments, the pH of the antibody formulation is about 4.5-6.5; in some embodiments, the pH is about 4.5-6.0; in some embodiments, the pH is about 5.0-6.0; in some embodiments, the pH is about 5.2-5.8; in some embodiments, the pH is about 5.5. In some embodiments, the pH of the antibody formulation is about 4.5, about 5.0, about 5.2, about 5.5, about 5.8, about 6.0, or a range between any two pH values, including endpoint values. In some embodiments, the buffer in the antibody formulation is a histidine buffer, a glutamine buffer, a sodium acetate buffer, a succinate buffer or a citrate buffer; in some embodiments, the buffer is a histidine buffer, a glutamine buffer and a sodium acetate buffer; in some embodiments, the buffer is a histidine buffer. In some embodiments, the concentration of the buffer is about 5-30 mM; in some embodiments, the concentration of the buffer is about 10-20 mM; in some embodiments, the concentration of the buffer is about 10 mM. In some embodiments, the buffer is a histidine buffer at about 10 mM. In some embodiments, the concentration of the histidine buffer is about 5 mM, about 10 mM, about 20 mM, about 30 mM, or a range between any two concentration values, including endpoint values.

In some embodiments, the antibody formulation comprises the anti-TNF-a antibody at about 5-130 mg/mL, the stabilizer at about 160-265 mM, the surfactant at about 0.05-0.5 mg/mL and the buffer at about 5-30 mM, and the pH of the antibody formulation is about 4.5-6.5. In some embodiments, the antibody formulation comprises the anti-TNF-a antibody at about 90-110 mg/mL, the stabilizer at about 210-240 mM, the surfactant at about 0.1-0.3 mg/mL and the buffer at about 10-20 mM, and the pH of the antibody formulation is 4.5-6.0. In some embodiments, the antibody formulation comprises the anti-TNF-a antibody at about 90-110 mg/mL, trehalose at about 210-240 mM, a polysorbate 80 at about 0.1-0.3 mg/mL and a histidine buffer at about 10-20 mM, and the pH of the antibody formulation is 4.5-6.0. In some embodiments, the antibody formulation comprises the anti-TNF-a antibody at about 100 mg/mL, the trehalose at about 225 mM, the polysorbate 80 at about 0.2 mg/mL and the histidine buffer at about 10 mM, and the pH of the antibody formulation is 5.0-5.8. In some embodiments, the antibody formulation comprises the anti-TNF-a antibody at about 100 mg/mL, arginine hydrochloride at about 10 mM to about 142 mM, the polysorbate 80 at about 0.2 mg/mL and the histidine buffer at about 10 mM, and the pH of the antibody formulation is 5.0-5.8. In some embodiments, the antibody formulation comprises the anti-TNF-a antibody at about 100 mg/mL, glycine at about 333 mM, the polysorbate 80 at about 0.2 mg/mL and the histidine buffer at about 10 mM, and the pH of the antibody formulation is 5.0-5.8. In some embodiments, the antibody formulation comprises the anti-TNF-a antibody at about 100 mg/mL, methionine at about 34 mM, the polysorbate 80 at about 0.2 mg/mL and the histidine buffer at about 10 mM, and the pH of the antibody formulation is 5.0-5.8. In some embodiments, the anti-TNF-a antibody is Simponi^{®} (golimumab) or biological analogs thereof (e.g., BOW100, ONS-3035 or Antibody A).

In some embodiments, the antibody formulation comprises Antibody A at about 100 mg/mL, the histidine hydrochloride at about 1.72 mg/mL, the histidine at about 0.28 mg/mL, trehalose dihydrate at about 85 mg/mL and the polysorbate 80 at about 0.2 mg/mL, and the pH of the antibody formulation is about 5.5.

In some embodiments, the amount of acidic antibody variants in the anti-TNF-a antibody formulation is no more than about 40%, no more than about 35% and no more than about 30%. In some embodiments, the amount of acidic antibody variants in the anti-TNF-a antibody formulation is about 15%, about 16%, about 17%, about 18%, about 19%, 20%, about 21%, about 22%, about 23%, about 24%, about 25%, about 26%, about 27%, about 28%, about 29% or about 30%, or a range between any two concentration values, including endpoint values.

In some embodiments, the anti-TNF-a antibody formulations provided herein have an acidic variant amount of no more than 30% after high temperature (40 °C ± 3 °C) storage for 1 week, 2 weeks, 3 weeks or 4 weeks.

The present invention provides liquid aqueous pharmaceutical formulations comprising antibodies suitable for therapeutic use, primarily for the treatment of diseases caused by TNF-α. The pharmaceutical formulation is easy to administration, has a high protein concentration and supports subcutaneous injection. In some embodiments, the pharmaceutical formulation has the effect of enhancing stability and keeping a low level of deamidation of the antibody. In other embodiments, the formulations of the present invention are stable after at least 5 freeze-thaw cycles. In other embodiments, the formulations of the present invention remain stable after high temperature (40 °C) storage for 15 days. In other embodiments, the antibody is a human TNF-α antibody. In other embodiments, the antibody is Antibody A.

### Administration route

The antibody formulation of the present invention may be administered by a variety of methods known in the art. In some embodiments, the antibody formulation may be administrated by subcutaneous injection. In other embodiments, the antibody formulation may be administrated by intravenous injection, intravenous infusion, or perfusion. It will be appreciated by those skilled in the art that administration route will vary depending on the desired result.

In some embodiments, the antibody formulation is administered to the patient by subcutaneous injection. In some embodiments, the antibody is administered at a dose of 20 mg to 100 mg; in some embodiments, the antibody is administered at a dose of about 50 mg. The antibody is administrated once every 1 week to every 6 weeks; in some embodiments, the antibody is administrated once every 4 weeks. In some embodiments, the antibody formulation is administered to the patient by subcutaneous injection once every 4 weeks or 1 month, and administration amount of the anti-TNF-a antibody is 50 mg.

In some embodiments, the patient is a patient with psoriatic arthritis, rheumatoid arthritis, ankylosing spondylitis, ulcerative colitis and the like.

In some embodiments, the patient is a patient with ulcerative colitis, and the anti-TNF-a antibody is firstly administrated at a dose of about 200 mg, about 100 mg in week 2, and then about 100 mg once every 4 weeks.

### TNF-α related diseases

The term "TNF-α related diseases" as used herein includes that the presence of TNF-α in a subject having the disease has been shown or TNF-α is suspected to be responsible for the pathophysiology of the disease or to worsen the disease. The TNF-α related diseases include, but are not limited to, chronic inflammatory diseases such as psoriatic arthritis, rheumatoid arthritis, ankylosing spondylitis, ulcerative colitis and the like.

### EXAMPLES

### Example 1: Amino acid sequence of the Antibody A protein, and expression and purification thereof

### 1. Amino acid sequence of Antibody A protein

The anti-TNF-a human antibody (Antibody A) in the formulation of the present invention is a recombinant anti-tumor necrosis factor α fully human monoclonal antibody with the molecular weight of about 150 kDa, is an IgG1 antibody, and consists of two IgG1 heavy chains and two κ light chains. Each heavy chain with a molecular weight of 51 kDa contains 456 amino acids, and the amino acid sequence thereof is set forth in SEQ ID NO: 1 in a sequence table; each light chain with a molecular weight of 24 kDa contains 215 amino acids, and the amino acid sequence thereof is set forth in SEQ ID NO: 2 in the sequence table. Antibody A protein may be expressed in CHO cells by recombinant DNA techniques and obtained after purification by a series of standard chromatographic steps.

### 2. Expression and purification of Antibody A protein

An expression vector containing an antibody gene was constructed by referring to molecular cloning in molecular biology (Molecular Biology of the Gene, 2014), and then Antibody A was expressed using CHO-K1 cell (ATCC CCL61) as a host cell (Wood et al, Journal of Immunology. 145:3011 (1990)). The process of constructing stable cell lines was described below: the host cells were grown in suspension in CD CHO AGT^{™} medium (Gibco, CA, 12490017); the host cells in the logarithmic phase were centrifuged, and resuspended in fresh CD CHO CD CHO AGT^{™} medium; 0.8 mL of the above cell suspension was taken and added to a cuvette; then 40 µg each of two types of double digested plasmids containing the light and heavy chain-encoding genes (pre-constructed expression vectors containing antibody genes, using plasmids pFUSE-CLIg and pFUSE-CHIg, Invivogen Inc., Cat# pfuse2-hclk and pfuse-hchg1) was added; the cells and plasmids were well mixed. Transformation was performed with a Bio-rad electrotransfer apparatus (Bio-rad, Gene PulserXcell). The electroporated cells were immediately resuspended in CD CHO AGT^{™} medium preheated at 37 °C and added to a 96-well plate at 0.1 mL per well, and an equivalent amount of screening medium (CD CHO AGT^{™} medium + 50 µM methionine iminosulfone (MSX, purchased from Sigma-Aldrich, 76078)) was added after 2-3 days. The expression level of the antibody in the cell culture supernatant in the 96-well plate was determined using a multifunctional microplate reader (Molecular Devices, Spectramax M4). The clone cells with higher expression level were transferred from the 96-well plate to a 24-well plate containing a screening medium for culture, the cells were transferred to a 6-well plate containing the screening medium for culture after the cells grew for 7 days, the antibody yield of the cells was determined, and the clone cells with high antibody expression were transferred to shake flasks. 5-8 clones with the highest antibody expression were screened out for subcloning and the screening process was repeated, and finally 1 engineered cell with the highest antibody expression was obtained. A bioreactor was adopted to perform fermentation culture on the cells. The culture medium was centrifuged at a low speed to separate the cells from the medium, and the supernatant was further cleared by centrifugation at a high speed. The primary purification was performed by affinity chromatography (gel medium, MabSelectSuRe LX (GE)) using a protein purification liquid chromatography system (GE Healthcare, AKTA PROCESS 10 MM). The purification could achieve the expected purpose, effectively captured target proteins and simultaneously effectively removed most of impure proteins, and the purity of the target proteins was detected by SDS-PAGE using Power Pac Basic (BIO-RAD) adopting a method (introduced in Operation Guide on Practical Molecular Biology, People's Medical Publishing House Co., Ltd, 2003), wherein the purity reaches more than 90%.

After primary purification, further separation and purification were performed using a protein purification liquid chromatography system (the same as above) and adopting anion (Capto Q medium, GE) and cation (Capto S ImpAct medium, GE) exchange chromatography, impurities such as endotoxin, HCP, DNA and the like were further removed, and the amount of SEC monomer was over 99% through size-exclusion chromatography (SEC) detection (the same as above).

### Example 2: Concentration screening of surfactant in antibody formulations

Antibody A formulation samples containing different surfactant (polysorbate 80) concentration were prepared separately using the Antibody A prepared in Example 1:
B1: containing no surfactant;
B2: containing 0.1 mg/mL polysorbate 80;
B3: containing 0.2 mg/mL polysorbate 80;
B4: containing 0.4 mg/mL polysorbate 80;

Antibody A, stabilizers, buffers and solvents and concentrations thereof are shown in Table 1 below (example surfactant at 0.2 mg/mL in Table 1).

**Table 1. Ingredients in Antibody A formulation**

| Functions | Ingredients | Amounts | Concentrations |
|---|---|---|---|
| Active substance | Antibody A | 50.00 mg | 100 mg/ml |
| Stabilizers | Trehalose dihydrate | 42.50 mg | 85 mg/ml |
| Surfactant | Polysorbate 80 | 0.10 mg | 0.20 mg/ml |
| Buffers | Histidine | 0.14 mg | 0.28 mg/ml |
| | Histidine hydrochloride | 0.86 mg | 1.72 mg/ml |
| Solvent | Water for injection | fill to 0.5 mL | - |

The 4 formulations B1-B4 were diluted with 0.9% sodium chloride solution at a ratio of 1:200, mixed well by gentle shaking, and after standing for 2 h, insoluble particles measurement was performed using a liquid particle counter (Beckman Coulter, HIAC 9703+) according to the method recommended by manufacturer. The results are shown in Table 2.

**Table 2. Results of measuring the number of insoluble particles after dilution of Antibody A formulations containing different surfactant concentrations**

| Samples | First measurement on partical number | | Second measurement on partical number | | Third measurement on partical number | | Mean value (number/mL) | |
|---|---|---|---|---|---|---|---|---|
| | ≥10 µm | ≥25 µm | ≥10 µm | ≥25 µm | ≥10 µm | ≥25 µm | ≥10 µm | ≥25 µm |
| Control-water | 1 | 0 | 1 | 0 | 0 | 0 | 1 | 0 |
| Control-0.9% sodium chloride solution | 1 | 0 | 0 | 0 | 1 | 0 | 1 | 0 |
| B1 | 99 | 2 | 115 | 4 | 90 | 6 | 101 | 4 |
| B2 | 69 | 1 | 86 | 2 | 80 | 1 | 78 | 1 |
| B3 | 31 | 0 | 29 | 1 | 27 | 1 | 29 | 1 |
| B4 | 63 | 1 | 50 | 2 | 75 | 3 | 63 | 2 |

As shown in the above Table, the number of particles of the antibody formulation without a surfactant (B1) was significantly higher than that of the other formulations (B2-B4), and the number of particles of the antibody formulation with a surfactant added (B2-B4) was significantly reduced.The amount of surfactant added also had an effect on the number of particles, and among formulations B2-B4 to which different amounts of surfactant were added, the number of particles of formulations (B3 and B4) containing polysorbate 80 at concentrations of 0.2 mg/mL and 0.4 mg/mL respectively, in particular, the number of particles of ≥ 10 µm was significantly lower than that of formulation (B2) containing polysorbate 80 at 0.1 mg/mL, and the number of particles of antibody formulation B3 containing polysorbate 80 at 0.2 mg/mL was the lowest.

### Example 3: Buffer screening

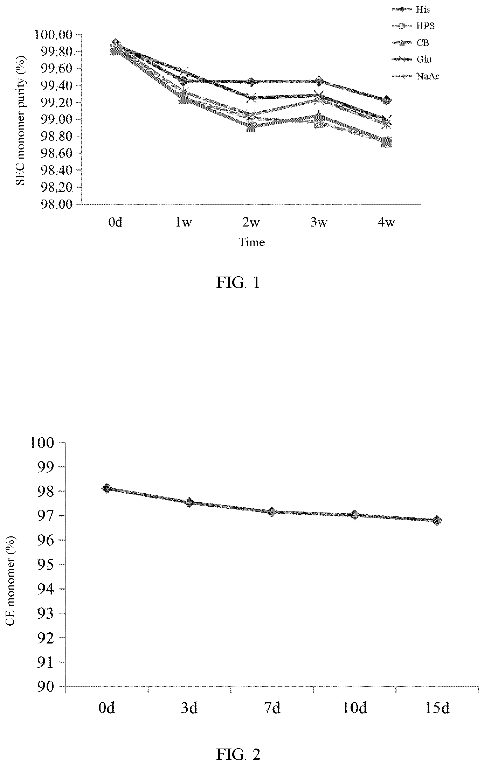

In order to find a suitable formulation buffer, a number of formulations containing different buffers were prepared (see Table 3) due to the different buffering capacities of the different buffers. The ingredients in the formulations, except for the buffer, and amounts thereof were the same as those shown in Table 1, and the pH of each formulation was 5.5. The formulations were stored under temperature 40 °C for 4 weeks. After 4 weeks, each formulation was subjected to size-exclusion chromatography (SEC) using high performance liquid chromatography (Agilent, 1260 Infinity) by a method (disclosed in Characterization of the size variants of a recombinant humanized monoclonal antibody (rhumAb1). Acta PharmaceuticaSinica, 2016:1897-1905), and the results are shown in FIG. 1.

**Table 3. Multiple formulations containing different buffers**

| Formulatio n number | Buffers | Ingredients |
|---|---|---|
| 1 | Succinate buffer (HPS) | Succinic acid: 1.18 g/L |
| 2 | Citrate buffer (CB) | Citric acid monohydrate: 0.599 g/L, |
| | | sodium citrate dihydrate: 2.103 g/L |
| 3 | Histidine buffer (His) | Histidine: 0.28 g/L, histidine |
| | | hydrochloride: 1.72 g/L |
| 4 | Glutamine buffer (Glu) | Glutamic acid: 1.47 g/L |
| 5 | Sodium acetate buffer | Glacial acetic acid: 0.06 g/L, sodium |
| | (NaAc) | acetate trihydrate: 1.23 g/L |

As shown in FIG. 1, the SEC monomer purity differences of 5 formulations as shown in Table 3 are significant, and the protection effect of His buffer on antibody is the best, followed by Glu and NaAc buffers.

### Example 4: pH screening of antibody formulations

pH is a critical factor in maintaining antibody stability. A particular antibody can be stable only at a particular pH. Therefore, in order to study that the antibody of the present invention has good stability under which pH conditions, a pH screening was performed. 6 histidine buffers with different pH values were prepared, and the pH values ranged from 4.5 to 6.5. The specific groupings were: sample 1, pH 4.5; sample 2, pH 5.0; sample 3, pH 5.5; sample 4, pH 5.8; sample 5, pH 6.0; sample 6, pH 6.5. The ingredients of the formulation and amounts thereof, except for the buffer, are shown in Table 1.

Comparative experiment was performed on the above 6 groups of samples at high temperature (40 °C). Samples at 1, 2, 3, and 4 weeks of time were taken and detected for antibody stability by ion exchange chromatography (IEC) using high performance liquid chromatography (Agilent, 1260 Infinity) by the method (disclosed in Harris R J et al, Identification of multiple sources of charge heterogeneity in a recombinant antibody. Journal of chromatography. B, Biomedical sciences and applications, 2001, 752(2):233-245), and the detection results are shown in Table 4 below.

**Table 4. Comparison results of samples with different pH ranges at high temperature**

| Time point | Sample number | IEC-HPLC (%) | | |
|---|---|---|---|---|
| | | Acidic region | Main peak | Basic peak |
| Week 1 | 1 | 30.97 | 50.54 | 18.49 |
| | 2 | 29.39 | 52.74 | 17.87 |
| | 3 | 31.45 | 50.05 | 18.50 |
| | 4 | 30.18 | 50.45 | 19.37 |
| | 5 | 34.25 | 46.68 | 19.07 |
| | 6 | 51.76 | 32.54 | 15.70 |
| Week 2 | 1 | 38.34 | 48.23 | 13.43 |
| | 2 | 36.02 | 50.21 | 13.78 |
| | 3 | 39.03 | 48.99 | 11.98 |
| | 4 | 36.31 | 49.71 | 13.99 |
| | 5 | 43.70 | 44.02 | 12.28 |
| | 6 | 66.64 | 26.25 | 7.11 |
| Week 3 | 1 | 40.58 | 43.22 | 16.20 |
| | 2 | 36.65 | 45.80 | 17.55 |
| | 3 | 37.97 | 44.79 | 17.25 |
| | 4 | 39.28 | 43.42 | 17.30 |
| | 5 | 44.05 | 38.00 | 17.95 |
| | 6 | 65.88 | 23.68 | 10.44 |
| Week 4 | 1 | 48.32 | 41.07 | 10.61 |
| | 2 | 47.32 | 42.05 | 10.62 |
| | 3 | 46.00 | 43.95 | 10.05 |
| | 4 | 47.03 | 40.96 | 12.01 |
| | 5 | 55.29 | 32.37 | 12.33 |
| | 6 | 77.22 | 18.59 | 4.20 |

It can be seen from IEC-HPLC detection data that the amounts of IEC-HPLC main peak and acidic variant of the 6 samples are obviously different, in which the result of sample 6 is the worst, the result of sample 5 is slightly better than that of sample 6, and the result of other 4 samples are slightly different, indicating that the antibody formulation of the Antibody A has better IEC stability under the condition that the pH is less than 6.0, wherein the main peak of the Antibody A sample is the highest and the acidic variant is the lowest at pH 5.5, indicating that the stability of the Antibody A formulation is the best at pH 5.5.

### Example 5: Stabilizer screening

In order to screen the stabilizer for the antibody formulation of the present invention, antibody formulations containing different stabilizers were prepared. The stabilizers used and the amount thereof were shown in Table 5, and the other ingredients in the formulation, except for the stabilizer, were the same as those shown in Table 1. Each formulation was stored at 25 °C under illumination condition (light intensity 4000 1x) in a incubator for 2 weeks. After 0 days, 7 days and 2 weeks of illumination, each formulation was detected for antibody stability by ion exchange chromatography (IEC), and the detection results are shown in Table 6.

**Table 5. Formulations containing different stabilizers**

| Formulation sample number | | Stabilizers |
|---|---|---|
| | 1 | 230 mM Sorbitol |
| | 2 | 230 mM Mannitol |
| | 3 | 154 mM Sodium chloride |
| | 4 | 217 mM Proline |
| | 5 | 234 mM Sucrose |
| | 6 | 142 mM Arginine hydrochloride |
| | 7 | 10 mM Histidine hydrochloride |
| | 8 | 333 mM Glycine |
| | 9 | 34 mM Methionine |
| | 10 | 225 mM Trehalose dihydrate |

**Table 6. Stability data for formulation samples containing different stabilizers under illumination condition**

| Time | Formulation number | | IEC-HPLC (%) | | |
|---|---|---|---|---|---|
| | | | Acidic peak | Main peak | Basic peak |
| Day 0 | | 1 | 30.94 | 52.64 | 16.42 |
| | | 2 | 31.12 | 52.54 | 16.34 |
| | | 3 | 31.15 | 52.28 | 16.57 |
| | | 4 | 31.06 | 52.50 | 16.43 |
| | | 5 | 30.98 | 52.68 | 16.34 |
| | | 6 | 31 | 52.70 | 16.30 |
| | | 7 | 31.15 | 52.31 | 16.54 |
| | | 8 | 30.87 | 52.70 | 16.42 |
| | | 9 | 30.97 | 52.61 | 16.41 |
| | | 10 | 30.96 | 52.74 | 16.29 |
| Day 7 | | 1 | 30.82 | 52.21 | 16.96 |
| | | 2 | 29.78 | 53.27 | 16.95 |
| | | 3 | 30.42 | 52.42 | 17.16 |
| | | 4 | 29.59 | 52.82 | 17.59 |
| | | 5 | 32.25 | 51.87 | 15.86 |
| | | 6 | 29.44 | 52.81 | 17.75 |
| | | 7 | 29.56 | 52.49 | 17.95 |
| | | 8 | 30.3 | 52.48 | 17.23 |
| | | 9 | 30.44 | 52.34 | 17.23 |
| | | 10 | 26.3 | 51.37 | 22.32 |
| Day 14 | | 1 | 51.54 | 39.86 | 8.59 |
| | | 2 | 50.87 | 40.12 | 9.01 |
| | | 3 | 50.73 | 39.79 | 9.47 |
| | | 4 | 40.19 | 42.02 | 17.80 |
| | | 5 | 45.01 | 38.06 | 16.94 |
| | | 6 | 49.75 | 41.75 | 8.50 |
| | | 7 | 34.81 | 46.02 | 19.18 |
| | | 8 | 39.29 | 43.17 | 17.55 |
| | | 9 | 39.44 | 43.03 | 17.53 |
| | | 10 | 29.01 | 51.94 | 19.05 |

It will be appreciated by those skilled in the art that the acidic variant of an antibody increases gradually with the increase of the storage time, and that an excessively high acidic variant has an adverse effect on the activity of the antibody. The interconversion of the acidic variant and the main peak is manifested as a change in the acidic variant and the main peak. In this experiment, as shown in Table 6 above, it can be seen from the amounts of IEC acidic variant and main peak that after 2 weeks of storage, the acidic peak amount of the formulation sample 10 increases slightly, and the main peak amount decreases minimally, indicating that the formulation sample 10 has good stability after 2 weeks of storage. The stabilizer adopted by the formulation sample 10, i.e., trehalose, has a good protective effect on the stability of the formulation samples under illumination condition.

In addition, after 2 weeks of storage, the main peak amount of the formulation sample 7 decreases slightly, and the acidic variant amount increases slightly, indicating that the formulation sample 7 is also better in stability after 2 weeks of storage. It means that the arginine hydrochloride has better protective effect on the stability of formulation samples. It can be seen from the data for formulation samples 8 and 9 that glycine and methionine also have a better protective effect on the formulation samples.

### Example 6: Preparation of Antibody A formulation

Based on the experiments as described in Examples 2-5 above, an Antibody A formulation of the present invention was prepared with polysorbate 80 at 0.2 mg/mL as a surfactant, His buffer as a buffer and trehalose as a stabilizer, and the pH of the formulation was 5.5.

The ingredients in the Antibody A formulation of the present invention comprises trehalose, polysorbate 80, histidine, histidine hydrochloride, Antibody A and water for injection.

The pharmaceutical formulation of the present invention was prepared by the following:
(1) weighing 850 g of trehalose dihydrate, 2 g of polysorbate 80, 2.8 g of histidine, 17.2 g of histidine hydrochloride, and 10 L of water for injection;
(2) dissolving the weighed ingredients (the order of adding trehalose dihydrate, polysorbate 80, histidine and histidine hydrochloride was not limited) in 9 L of water for injection to prepare a solvent system;
(3) mixing solvent system prepared above with the Antibody A concentrate containing a 1000 g of total protein under stirring, and finally quantifying to 10 L with water for injection to obtain the primary formulation of the present invention;
(4) sterilizing the primary formulation prepared above by passing through a hydrophilic poly(vinylidene fluoride) or polyethersulfone filter with a pore diameter of 0.22 µm, and filtering the formulation into sterile containers; and
(5) packaging the formulation after sterilization for use, for example, in a pre-filled syringe.

The volume of the formulations prepared above and the weight of ingredients can be scaled up or down, for example, 8 L, 7 L, 6 L, 5 L of formulations include 80%, 70%, 60%, 50% of the listed weights, respectively.

### Example 7: Stability of Antibody A formulation of the present invention at high temperature (40 °C)

The stability of the Antibody A formulation prepared in Example 6 at high temperature was further detected. The Antibody A formulation prepared above was stored at high temperature (40 °C) for 15 days, and then detected by capillary electrophoresis (CE) using a capillary electrophoresis apparatus (Agilent, CE 7100) by the method (disclosed in, for example, Chen G et al, Characterization of glycoprotein biopharmaceutical products by Caliper LC90 CE-SDS gel technology. Methods Mol Biol, 2013, 988(988): 199-209), and the detection results are shown in FIG. 2.

As shown in FIG. 2, the CE main peak of antibody of the Antibody A formulation stored for 15 days at high temperature (40 °C) decreases slightly to 96.79% from 98.11%, and is higher than 95%, indicating that the antibody in the Antibody A formulation has a good stability, and the formulation of the present invention has excellent protection effect on the Antibody A.

### Example 8: Freeze-thaw stability of Antibody A formulations of the present invention

Three Antibody A formulations were prepared at a low antibody concentration (LC, 5 mg/mL), at an antibody concentration (MC, 50 mg/mL), and at a high antibody concentration (HC, 100 mg/mL), respectively, as described above in Example 6. Each formulation was stored in a refrigerator at -80 °C, then taken out, placed at 25 °C for dissolving, and subjected to freeze-thaw for 5 times; the stability of the formulations after 5 times of freeze-thaw was determined. After 5 times of freeze-thaw, three formulations were diluted 1 × 10⁵, 1 × 10⁶ and 1 × 10⁶ times, respectively, and subjected to ELISA detection using a multifunctional microplate reader (Molecular Devices, Spectramax M4) by the method (disclosed in Wang Yuxiao et al., Isolation, purification and identification of anti-TNF-a human single-chain antibody, Journal of Immunology, 2008(6):700-702). The antibody was loaded into 5 wells for each dilution and the determined recovery and coefficient of variation (CV) are listed in Table 7.

**Table 7. Freeze-thaw result of Antibody A formulations at different antibody concentrations**

| Formulation samples | | LC | MC | HC |
|---|---|---|---|---|
| Antibody concentration (mg/mL) | | 5 | 50 | 100 |
| Detection results | | 58.34 | 54.46 | 120.23 |
| | (n = 5) | 57.89 | 52.12 | 114.37 |
| | (ng/mL) | 55.51 | 50.73 | 97.46 |
| | | 48.67 | 50.24 | 92.58 |
| | | 47.94 | 46.07 | 95.66 |
| Mean × dilution factor (mg/mL) | | 5.367 | 50.72 | 104.06 |
| Standard deviation | | 0.50 | 3.07 | 12.39 |
| Recovery (%) | | 107.3 | 101.4 | 104.06 |
| CV (%) | | 9.3 | 6.1 | 11.9 |

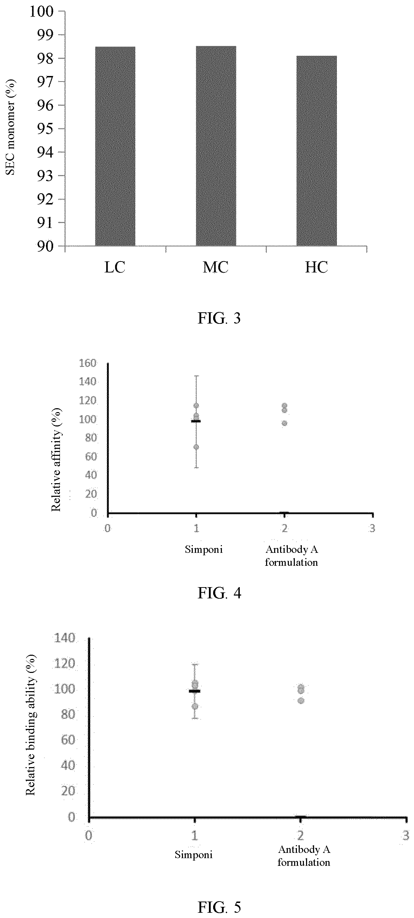

In addition, SEC detection was performed on each dilution of the formulation with different antibody concentrations using high performance liquid chromatography (Agilent, 1260 Infinity) by the method disclosed by Zhao J et al (the same as above), and the results are shown in FIG. 3.

The results show that after 5 times of freeze-thaw, the concentration recovery (%) of the antibody formulation with the high, medium and low levels of the Antibody A ranges from 101.4% to 107.3%, and the variation coefficient ranges from 6.1% to 11.9%; the SEC purity is above 98%.The antibody formulation with different antibody concentrations has stable quality after 5 times of repeated freeze-thaw, and has good protection effect on the Antibody A.

### Example 9: Study on microorganisms

The study on microorganisms in Antibody A formulations of the present invention was further performed. Antibody A formulations were prepared as described in Example 6 above and subjected to microorganism study to determine whether the formulations could support microorganism growth. The above-mentioned sterile formulations were inoculated with the microorganisms (*Escherichia coli* (CICC-10003), *Staphylococcus aureus* (CICC-10306), *Pseudomonas aeruginosa* (CICC-10351) and *Bacillus cereus* (CICC-10352)) in an amount of 100 cfu/mL, and after 14 days of storage at room temperature of 20-25 °C, the formulations inoculated with the microorganisms were detected for the total microorganism growth in the inoculated containers. The number of microorganisms and the change in turbidity (measured by a turbidity meter (HACH, 2100AN 4700100)) were mainly evaluated by a microscope (Carl Zeiss, Axiovert 25 662798), the detection results are shown in Table 8 below, wherein no change in turbidity indicates no microorganism grows. It can be seen from the data shown in Table 8 below that no microorganism was observed under a microscope after 14 days of storage at room temperature from 20-25 °C, and that the turbidity of the formulation did not change, so the formulation of the present invention did not support microorganism growth.

**Table 8. Detection results of microorganisms in Antibody A formulations**

| Detection index | Number of microorganisms cfu/mL | Turbidity/NTU |
|---|---|---|
| *Escherichia coli* | 0 | No change in turbidity |
| *Staphylococcus aureus* | 0 | No change in turbidity |
| *Bacillus cereus* | 0 | No change in turbidity |
| *Pseudomonas aeruginosa* | 0 | No change in turbidity |

### Example 10: Comparison in pharmacodynamics of Antibody A formulation with that of Simponi

The Antibody A formulation prepared above was subjected to pharmacodynamic studies *in vitro* and *in vivo* animal models. *In vitro* experiment mainly performed pharmacodynamic detections on the following aspects: ability to bind to TNF-α, affinity with TNF-α, inhibition on the biological activity of TNF-α.

The affinity of the Antibody A of the present invention to TNF-α was verified. The specific experimental method was as follows: the Antibody A formulation prepared above in Example 6 was first diluted to 2 µg/mL with PBS; TNF-α (10602-HNAE, Sino Biological Inc.) was diluted to 20 nM with PBS and 2-fold gradient dilution was performed to obtain 20, 10, 5, 2.5, 1.25, 0.625, 0.3125 nM TNF-α dilutions. Detection was
performed using BIAcore (GE Healthcare, T200) with Protein A chip (GE Healthcare, 29127556), and 2 µg/mL of Antibody A formulation was passed through the experimental channel (Fc2, Fc4) at a flow rate of 10 µL/min, and the amount of capture was about 200RU for 10 s; then the flow rate was adjusted to be 30 µL/min, TNF-α dilutions with different concentrations sequentially passed through the surfaces of the experiment flow paths (Fc2 and Fc4) and the reference flow paths (Fc1 and Fc3), the binding time was 120 s, the dissociation time was 600 s, and finally the chip was subjected to regeneration by the Glycine 1.5 (Shanghai Aladdin Bio-Chem Technology Co., Ltd, A110752) for 60 s and entered the next cycle. Experiment results were analyzed using data analysis software Evaluation software 3.1, the sensing signals acquired by the sample experiment flow path passes through reference flow paths, blank double subtraction was performed on samples, kinetic fitting was performed using 1:1 model to obtain the kinetic parameters (ka: association rate; kd: dissociation rate; kD: dissociation equilibrium constant) of each sampls and TNF-α. The same experiment was performed with commercially available Simponi. It can be seen from the results that the affinity constant of the Antibody A formulation prepared by the present invention binding to TNF-α is about 60 pM (the Antibody A formulation is 56.6 pM-67.4 pM, Simponi is 50.9 pM-72.1 pM), indicating that the anti-TNF-a antibody formulation of the present invention has strong affinity with TNF-α.

The affinities of Antibody A standard (hereinafter referred to as standard S with protein at 110.4 mg/mL and 1.980×10⁵ U/mg active Antibody A) and Simponi were detected using the same method as above, and the relative affinities of Antibody A of the present invention and commercially available Simponi with respect to standard S were further calculated. It can be seen from the results that the relative affinities of the Antibody A of the present invention and Simponi binding to TNF-α are substantially identical, as shown in FIG. 4.

The binding ability of the Antibody A formulation of the present invention to TNF-α was also detected (see Wang Yuxiao et al., Isolation, purification and identification of anti-TNF-a human single-chain antibody, Journal of Immunology, 2008(6):700-702) and the inhibitory effect on TNF-α biological activity was detected (see Trost L C et al, A cytotoxicity assay for tumor necrosis factor employing a multiwell fluorescence scanner. Analytical Biochemistry, 1994, 220(1): 149-153). It can be seen from the results that the Antibody A formulations of the present invention have an EC50 for binding TNF-α of about 8 ng/mL and an EC50 for inhibiting the biological activity of TNF-α of about 6 ng/mL.

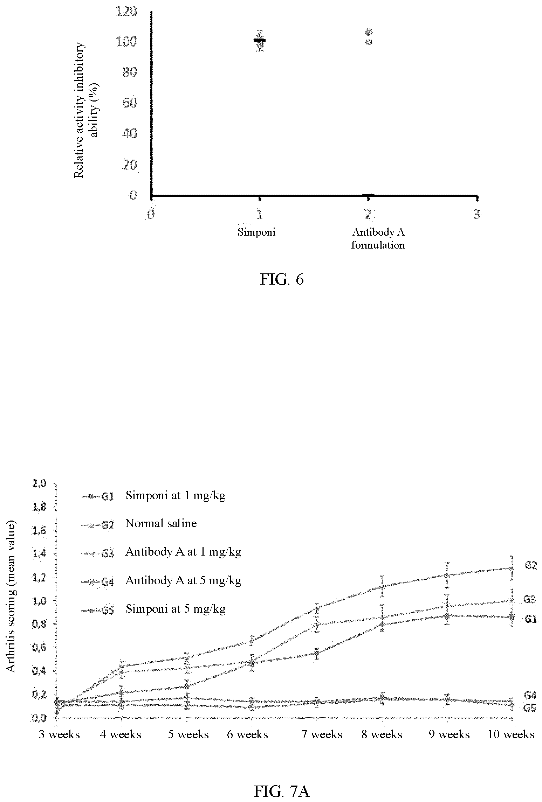

The relative binding ability and relative activity inhibition ability of the Antibody A formulation of the present invention to TNF-α relative to the standard S were further calculated. The relative binding ability and relative activity inhibition ability of the commercially available Simponi to TNF-α relative to the standard S were detected by the same methods as above. The results are shown in FIGs. 5 and 6. As shown in FIGs. 5 and 6, the binding ability of the Antibody A formulation of the present invention and Simponi to TNF-α, and the inhibitory effect of the Antibody A formulation of the present invention and Simponi on the biological activity of TNF-α are substantially identical.

The *in vivo* pharmacodynamic evaluation on the Antibody A of the present invention was performed in a mouse model of human TNF transgenic spontaneous arthritis (Tg197, Biomedcode Hellas SA). The transgenic mice were divided into 5 groups, and 8 mice in each group were intraperitoneally injected with 1mg/kg Simponi (G1), normal saline (G2), 1 mg/kg Antibody A (G3), 5 mg/kg Antibody A (G4) and 5 mg/kg Simponi (G5). Injections were initiated on week 3, twice a week, and stopped on week 10. A control group of transgenic mice (4) was added and this group was sacrificed prior to the first injection, indicating the initial histopathological status. All experimental animals were sacrificed at week 10 and the ankle joints were collected for pathology evaluation. The scoring system used for the degree of arthritis lesion *in vivo* and the pathology scoring system used for the histopathological evaluation of ankle joints are shown as follow.

Scoring system for the degree of arthritis lesion *in vivo*

| **Arthritis scoring¹** | **Characteristics** |
|---|---|
| 0/no | No arthritis (normal appearance, able to attach to inverted or inclined surfaces of wire mesh or cage cover for a period of time, normal general mobility/hiding ability, maximum grip strength) |
| 0.5/mild | Onset of arthritis (mild swelling of joints, all other parameters are the |

| | |
|---|---|
| | same as above) |
| 1/mild to moderate | Mild to moderate arthritis (swelling of joints, inflammation of paws, all other parameters are the same as above) |
| 1.5/moderate | Moderate arthritis (swelling and deformation of the joints and paws + inward deformation of the distal toes, able to attach to inverted or inclined surfaces of wire mesh or cage cover, reduced general mobility and reduced grip strength) |
| 2/moderate to severe | Moderate to severe arthritis (severe swelling of joints, paws and toes, deformable joint-leg, unable to attach to inverted or inclined surfaces of wire mesh or cage cover, lack of general mobility, lack of grip strength, impaired climbing/eating, shaking when trying to move, but moving barely forward) |
| 2.5/severe | Severe arthritis (same as the characteristics in the group of scored 2 + deformable toes, impaired mouse mobility, trembling and unwilling to move) |
| 3/very severe | Very severe arthritis (joint stiffness and severely impaired mobility detected during flexion, mice dying, no longer trembling, and unable to easily rotate/turn over when falling to one side). |

Evaluation system for ankle joints histopathology

| **Histopathological scoring criteria for phenotypic scoring of ankle joint arthritis** | | |
|---|---|---|
| **Scoring ¹** | **Degree of arthritis** | **Criteria** |
| 0 | Normal | No pathological changes detected |
| 1 | Mild | Synovial hyperplasia and polymorphonuclear leukocyte infiltration occurred. Mild tendonitis may be present. |
| 2 | Moderate | Pannus and fibrous tissue formation and focal subchondral bone erosion |
| 3 | Moderate -sever e | Cartilage destruction and bone erosion |
| 4 | Severe | Extensive cartilage destruction, bone erosion and bone infrastructure loss |

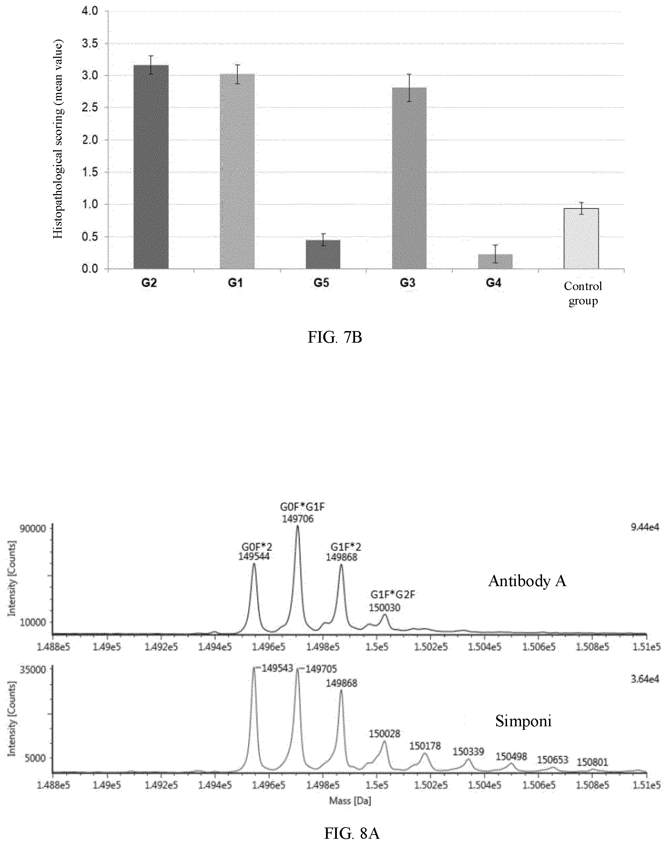

The results of the experiments are shown in FIGs. 7A and 7B, which indicates that the Antibody A of the present invention can inhibit the onset of arthritis in mice at both 1 mg/kg and 5 mg/kg, indicating that the Antibody A of the present invention has a therapeutic effect on the tg197 arthritis model.

### Example 11: Comparison in molecular weight and glycosylation of Antibody A of the present invention with that of commercially available Simponi

The complete and desugared molecular weights of Antibody A of the present invention and Simponi were detected using a high resolution mass spectrometer (Waters, Xevo G2-S QTof) according to the method recommended by the manufacturer.

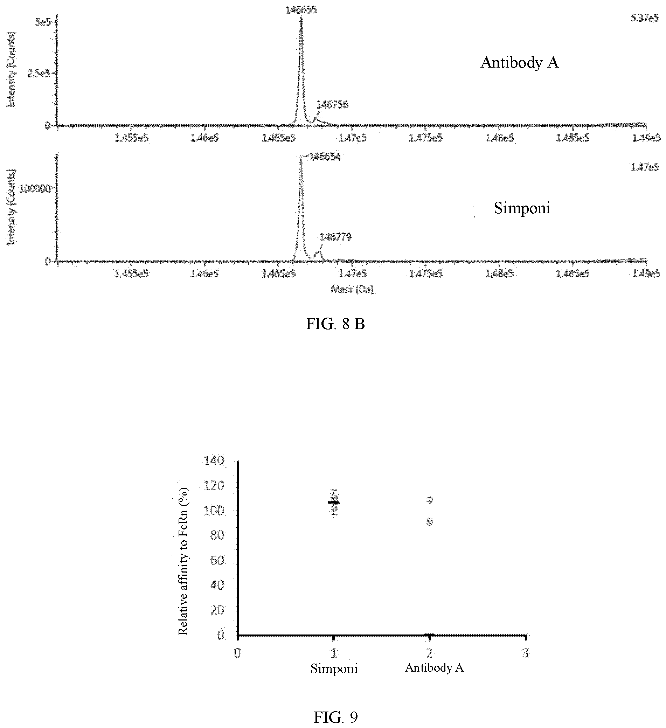

The Antibody A of the present invention and Simponi were subjected to deglycosylation treatment. The antibody was diluted to about 1 mg/mL with 50 mM of NH₄HCO₃ (pH 8.0), and N-glycosidase PNGaseF (Sigma; Cat# P7367-50 UN) was added to react at 37 °C for about 20 hours. Then, the Antibody A and the Simponi after deglycosylation were subjected to molecular weight detection. The results are shown in FIGs. 8A and 8B.

As shown by the comparison in the intact molecular weights of the Antibody A of the present invention (i.e., the anti-TNF-a antibody) with the Simponi in FIG. 8A, unlike the Antibody A of the present invention, Simponi has several peaks at a molecular weight of 150178 Da and at several larger molecular weights, and these several peaks disappear after the deglycosylation treatment (FIG. 8B). It can be concluded that the difference in molecular weights between the Antibody A of the present invention and Simponi result from the difference in NGNA glycosylation level. The Antibody A of the present invention has lower NGNA glycosylation level compared with the Simponi, so that the two have different molecular weights.

The amounts of NGNA in Antibody A and Simponi were detected (Spichtig V, Michaud J, Austin S. Determination of sialic acids in milks and milk-based products[J]. Analytical Biochemistry, 2010, 405(1):28-40) and the results were averaged for the three samples and are shown in Table 9 in mol/mol antibody, i.e., the molar amount of NGNA per mol antibody.

**Table 9. Amounts of NGAG in Antibody A and Simponi**

| | Sample 1 | Sample 2 | Sample 3 | Mean value |
|---|---|---|---|---|
| Antibody A | 0.0043 | 0.0043 | 0.0039 | 0.0042 |
| Simponi | 0.29 | 0.35 | 0.29 | 0.31 |

As for the amino acid sequences of the Antibody A and the Simponi, glycosylation modification mainly generates NGNA (N-glycolylneuraminic acid), and the NGNA glycosylation modification of TNF-α can cause immunogenicity and adverse reactions on a human body after the antibody enters the human body. Compared with the Simponi, the Antibody A has a lower NGNA glycosylation modification level and has a lower risk of adverse reaction on human body caused by the NGNA.

### Example 12: Comparison in Fc-terminal activity of Antibody A of the present invention with that of commercially available TNF-α Simponi

The binding activity of the Fc-terminal of an antibody to various cell surface receptors can affect various properties of the antibody, for example, binding of the Fc-terminal of an antibody to a cell surface FcRn can affect the half-life of the antibody in a human body, and binding of the Fc-terminal of an antibody to a cell surface FcyRIIIa can affect antibody-dependent cell-mediated cytotoxicity (ADCC) of the antibody.

This experiment verifies the affinity of the Antibody A of the present invention and Simponi binding to FcRn. The specific experiment method was as follows. FcRn-biotin (ACRO Biosystems, Cat# FCM-H82W4) was diluted to 1 µg/mL with PBS. An SA Biosensor (Fertebio, Cat# 18-5019) was used to immobilize receptor proteins in FcRn-biotin solution to a signal of approximately 1.2 nm. The Antibody A of the present invention and the Simponi were diluted to 200 nM with PBS, and then diluted with a 2-fold gradient until 3.125 nM. PBS, drug dilutions, 1 M MgCl₂ (pH 8.3) solution and PBST were added sequentially to the corresponding columns of a 96-well plate and the following steps were performed with an SA Biosensor immobilized with FcRn in an Octet Platform instrument (Fertebio, Octet QKe): baseline: baseline detection was performed in PBS for 60 s; association: binding was performed for 60 s in drug dilutions (200 nM, 100 nM, 50 nM, 25 nM, 12.5 nM, 6.25 nM, 3.125 nM) and sample blank (PBS); dissociation: dissociation was performed in PBS for 60 s; regeneration: regeneration was performed in 1 M MgCl₂ (pH 8.3) solution for 5 s; neutralization: neutralization was performed in PBST for 5 s. The cycle of regeneration and neutralization was performed 3 times. The data was processed and analyzed using Data Analysis 8.2 (Fertebio), and the sample data was fitted after sample blank (PBS) signal subtraction to obtain an affinity constant (KD). It can be seen from the results that the affinity constant of the Antibody A of the present invention binding to FcRn is about 7 nM (6.35 nM to 7.63 nM for Antibody A and 6.09 nM to 8.67 nM for Simponi).

The relative affinity of Antibody A of the present invention for binding to FcRn relative to standard S was calculated. The above experiments were performed on Simponi and the relative affinity of Simponi for binding to FcRn relative to standard S was calculated. The relative affinities of the Antibody A of the present invention and Simponi for binding to FcRn were substantially identical, and the results are shown in FIG. 9.

In addition, the affinity of the Antibody A of the present invention and Simponi for binding to FcRn was verified. FcyRIIIa has two forms, FcyRIIIa (F158) and FcyRIIIa (V158), which have been experimentally detected.

Specific experimental method for binding to FcyRIIIa (F158) were as follows. FcyRIIIa (F158)-biotin (ACRO Biosystems, Cat# CDA-H82E8) was diluted to 1 µg/mL. An SA Biosensor (Fertebio, Cat# 18-5019) was immobilize in FcyRIIIa (F158)-biotin dilution to a signal of approximately 0.6 nm. The Antibody A of the present invention and the Simponi were diluted to 500 nM with PBS, and then diluted with a 2-fold gradient until 7.812 nM. PBS, drug dilutions, 1 M MgCl₂ (pH 8.3) solution and PBST were added sequentially to the corresponding columns of a 96-well plate and the following steps were performed in an Octet Platform instrument (Fertebio, Octet QKe): baseline: baseline detection was performed in PBS for 60 s; association: binding was performed for 60 s in drug dilutions (500 nM, 250 nM, 125 nM, 62.5 nM, 31.25 nM, 15.62 nM, 7.812 nM) and sample blank (PBS); dissociation: dissociation was performed in PBS for 60 s; regeneration: regeneration was performed in 1 M MgCl₂ (pH 8.3) solution for 5 s; neutralization: neutralization was performed in PBST for 5 s. The cycle of regeneration and neutralization was performed 3 times. The data was processed and analyzed using Data Analysis 8.2, and the sample data was fitted after sample blank (PBS) signal subtraction to obtain an affinity constant (KD). It can be seen from the results that the affinity constant of the Antibody A of the present invention binding to FcyRIIIa (F158) is about 100 nM (110 nM to 116 nM for Antibody A and 93 nM to 111 nM for Simponi).

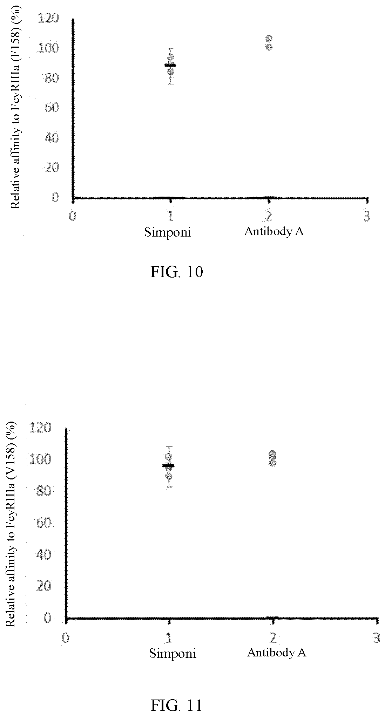

The relative affinity of Antibody A of the present invention for binding to FcyRIIIa (F158) relative to standard S was calculated. The above experiments were performed on Simponi, the relative affinity of Simponi for binding to FcyRIIIa (F158) relative to standard S was calculated, the relative affinities of Antibody A of the present invention and Simponi binding to FcyRIIIa (F158) were substantially identical, and the results are shown in FIG. 10.

Specific experimental method for binding to FcyRIIIa (V158) were as follows. FcyRIIIa (V158)-biotin (ACRO Biosystems, Cat# CDA-H82E9) was diluted to 1 µg/mL. An SA Biosensor (Fertebio, Cat# 18-5019) was immobilize in FcyRIIIa (V158)-biotin dilution to a signal of approximately 0.6 nm. The Antibody A of the present invention and the Simponi were diluted to 500 nM with PBS, and then diluted with a 2-fold gradient until 7.812 nM. PBS, drug dilutions, 1 M MgCl₂ (pH 8.4), PBST were sequentially added to the corresponding columns of the 96-well plate and the following steps were run in an Octet Platform instrument (Fertebio, Octet QKe): baseline: baseline detection in PBS for 60 s; association: binding for 60s in drug gradient dilutions (500 nM, 250 nM, 125 nM, 62.5 nM, 31.25 nM, 15.62 nM, 7.812 nM) and sample blank (PBS); disassociation: dissociation in PBS for 60 s; regeneration: regeneration in 1 M MgCl₂ (pH 8.4) for 5 s; neutralizing: neutralization was performed in PBST for 5 s. The cycle of regeneration and neutralization was performed 3 times. The data was processed and analyzed using Data Analysis 8.2, and the sample data was fitted after sample blank (PBS) signal subtraction to obtain an affinity constant (KD). It can be seen from the results that the affinity constant of the Antibody A of the present invention binding to FcyRIIIa (V158) is about 30 nM (29.2 nM to 40.8 nM for Antibody A and 20.1 nM to 42.2 nM for Simponi).

The relative affinity of Antibody A of the present invention for binding to FcyRIIIa (V158) relative to standard S was calculated. The above experiments were performed on Simponi, the relative affinity of Simponi for binding to FcyRIIIa (V158) relative to standard S was calculated, the relative affinities of Antibody A of the present invention and Simponi binding to FcyRIIIa (V158) were substantially identical, and the results are shown in FIG. 11.

### Example 13: Pharmacokinetic comparison in Antibody A of the present invention with the commercially available anti-TNF-a antibody (Simponi)

The purpose of this example is to study the pharmacokinetics of the Antibody A of the present invention and to provide a reference for future clinical rational drug use. Pharmacokinetic comparisons in Antibody A of the present invention with the commercially available anti-TNF-a antibody (Simponi) were performed simultaneously.

The study protocol was as follows:
6 groups of cynomolgus monkeys in total (purchased from Xingui Wild Animal Cultivation Co., Ltd. Yongfu county, 3-4.5 years old): low concentration of Antibody A of the present invention, high concentration of Antibody A of the present invention, low concentration of Simponi (Europe, purchased from Janssen Biologics B.V.), high concentration of Simponi (Europe), low concentration of Simponi (USA, purchased from Janssen Biologics B.V.) and high concentration of Simponi (USA), animals were grouped into 6 animals per group with half female and half male, the low concentration was 3 mg/kg, and the high concentration was 10 mg/kg. The animals were injected subcutaneously, whole blood was collected 1 h, 6 h, 24 h, 48 h, 3 d, 4 d, 5 d, 7 d and 9 d after administration, plasma was separated, and the concentration of each sample was analyzed.

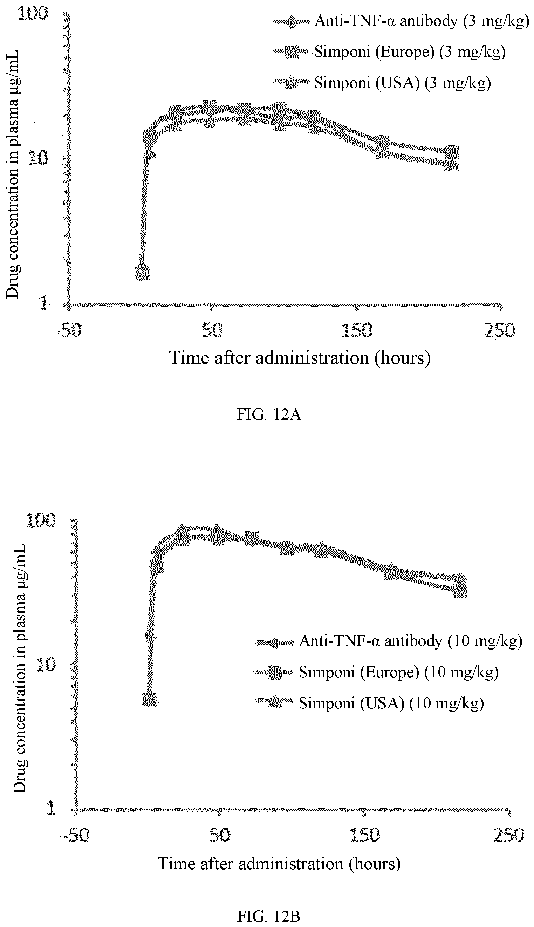

The determination method for the antibody amounts in the plasma sample comprises the following steps: the ELISA plate was coated with TNF-α protein (purchased from Sino Biological Inc., Cat#. 10602-HNAE) specifically binding to the anti-TNF-a antibody fragment to capture the antibody in the biological samples, then enzyme-labeled goat anti-human antibody (IgG-HRP, purchased from SIGMA, Cat#. A7164) was added, the reaction system was incubated at room temperature for about 1 hour to bind the enzyme-labeled goat anti-human antibody to the captured anti-TNF-a antibody, and finally a color-developing solution (purchased from BD Biosciences Inc., Cat#. 555214) was added to the plate and subjected to stand at room temperature for 15 minutes to develop color, the reaction was terminated with 2 mol/L sulfuric acid solution, and the OD value of each well was read at 450 nm using Spectramax M4 (Molecular Devices). A standard curve was obtained by four-parameter fitting the absorbance and concentration of the Antibody A standard sample S, and the obtained standard curve was used for quantifying the antibody concentration of the sample to be determined, which was determined on the same ELISA plate. Pharmacokinetic parameters of the drug were calculated using the WinNonLin 6.4 non-compartmental model (NCA) and mean concentration-time curves of the drug in plasma were generated and shown in FIG. 12.

It can be seen from the results that the pharmacokinetics of the Antibody A of the present invention and Simponi are similar in cynomolgus monkeys at a concentration ranging from 3 mg/kg to 10 mg/kg.

### Example 14: Stability of Antibody A formulations of the present invention

The long-term stability of the Antibody A formulation prepared as in Example 6 at 5 °C ± 3 °C was further studied. The Antibody A formulation prepared as above was stored at 5 °C ± 3 °C for 24 months in the absence of light, and the stability of the Antibody A was determined at month 0, month 3, month 6, month 9, month 12, month 18 and month 24, and the results are shown in Table 10.

The results in Table 10 show that the anti-TNF-a antibody is stored at 5 °C ± 3 °C for 24 months in the absence of light, and the quality attributes such as SEC, IEC, CE and the like only change slightly, and the anti-TNF-a antibody have good stability, indicating that the anti-TNF-a antibody in the anti-TNF-a antibody formulation of the present invention can be stable for at least 24 months.

**Table 10. Long-term stability results for Antibody A formulations at 5 °C ± 3 °C**

| Quality attributes | Month 0 | Month 3 | Month 6 | Month 9 | Month | Month | Month 24 |
|---|---|---|---|---|---|---|---|
| pH value | 5.4 | 5.6 | 5.5 | 5.5 | 5.4 | 5.5 | 5.5 |
| SEC-HPLC monomer amount (%) | 99.8 | 99.5 | 99.4 | 99.4 | 99.4 | 99.3 | 99.3 |
| SEC-HPLC polymer amount (%) | 0.2 | 0.4 | 0.5 | 0.5 | 0.5 | 0.6 | 0.6 |
| IEC-HPLC acidic variant amount (%) | 23.8 | 23.9 | 21.9 | 19.3 | 18.6 | 18.4 | 19.5 |
| IEC-HPLC main peak amount (%) | 53.8 | 55.1 | 56.8 | 56.9 | 56.2 | 57.1 | 54.4 |
| IEC-HPLC basic variant amount (%) | 22.4 | 21.0 | 21.3 | 23.8 | 25.2 | 24.5 | 26.1 |
| Non-reduced CE-SDS monomer amount (%) | 98.3 | 97.7 | 98.3 | 98.3 | 98.1 | 98.2 | 97.8 |
| Protein concentration (mg/mL) | 103.5 | 102.0 | 99.3 | 100.3 | 99.3 | 98.4 | 99.3 |
| Relative biological activity (%) | 100 | 98 | 101 | 99 | 94 | 93 | 101 |
| Relative binding activity (%) | 99 | 104 | 101 | 102 | 105 | 103 | 92 |

## Claims

1. An anti-TNF-a antibody formulation, comprising an anti-TNF-a antibody and a pharmaceutically acceptable carrier, wherein the anti-TNF-a antibody comprises a heavy chain with an amino acid sequence set forth in SEQ ID NO: 1 and a light chain with an amino acid sequence set forth in SEQ ID NO: 2, the pharmaceutically acceptable carrier comprises a stabilizer, a surfactant and a buffer, and the stabilizer is a sugar,arginine hydrochloride, glycine, methionine or a combination thereof.

2. The antibody formulation according to claim 1, wherein the pH of the antibody formulation is 4.5-6.5.

3. The antibody formulation according to claim 1 or 2, wherein the concentration of the anti-TNF-a antibody is 5-130 mg/mL.

4. The antibody formulation according to any one of claims 1 to 3, wherein the concentration of the stabilizer is 160-265 mM.

5. The antibody formulation according to any one of claims 1 to 4, wherein the surfactant is a polysorbate.

6. The antibody formulation according to any one of claims 1 to 5, wherein the concentration of the surfactant is 0.05-0.5 mg/mL.

7. The antibody formulation according to claim 1 or 2, wherein the buffer is one or more of a histidine buffer, a glutamine buffer, a sodium acetate buffer, a succinate buffer and a citrate buffer.

8. The antibody formulation according to any one of claims 1 to 7, wherein the concentration of the buffer is 5-30 mM.

9. An antibody formulation, comprising:
5-130 mg/mL of an anti-TNF-a antibody,
160-265 mM of a stabilizer,
5-30 mM of a buffer, and
0.05-0.5 mg/mL of a surfactant, and
a pH of 5.0-6.0.

10. An antibody formulation, comprising:
100 mg/mL of an anti-TNF-a antibody,
225 mM of a trehalose,
10 mM of a histidine buffer, and
0.2 mg/mL of a polysorbate 80 , and
a pH of 5.5.

11. A method for preparing the antibody formulation according to any one of claims 1 to 10, comprising:
weighing a stabilizer, a surfactant and a buffer;
dispersing the weighed ingredients in a liquid solvent for injection to prepare a solvent system; and
mixing the solvent system with the anti-TNF-a antibody.

12. An anti-TNF-a antibody pharmaceutical product, comprising the anti-TNF-a antibody formulation according to any of claims 1 to 10, wherein, preferably the antibody pharmaceutical product further comprises a pre-filled container.

13. Use of the antibody formulation according to any one of claims 1 to 10 or the antibody pharmaceutical product according to claim 12 in the preparation of a medicament for treating a TNF-α related disease.

14. A method for producing an anti-TNF-a antibody comprising a heavy chain with an amino acid sequence set forth in SEQ ID NO: 1 and a light chain with an amino acid sequence set forth in SEQ ID NO: 2, the method comprising:
culturing a CHO cell containing a nucleic acid or vector encoding the anti-TNF-a antibody such that the CHO cell expresses the anti-TNF-a antibody.

15. An anti-TNF-a antibody, comprising a heavy chain with an amino acid sequence set forth in SEQ ID NO: 1 and a light chain with an amino acid sequence set forth in SEQ ID NO: 2, wherein the molar ratio of the Neu5Gc sialic acid (NGNA) in the anti-TNF-a antibody to the anti-TNF-a antibody is no more than 0.1.
